(19) Europäisches Patentamt
European Patent Office
Office européen des brevets

(11) Veröffentlichungsnummer : **0 135 860**
**B1**

(12) # EUROPÄISCHE PATENTSCHRIFT

(45) Veröffentlichungstag der Patentschrift : **02.03.88**

(51) Int. Cl.[4] : **C 07 C147/14**, C 07 C147/10, A 61 K 31/11

(21) Anmeldenummer : **84110509.1**

(22) Anmeldetag : **04.09.84**

(54) **6-Sulfoxyphenolderivate, ihre Herstellung und ihre Verwendung als Cytoprotektiva; Verwendung von Salicylaldehydabkömmlingen als Cytoprotektiva.**

(30) Priorität : **10.09.83 DE 3332780**

(43) Veröffentlichungstag der Anmeldung : **03.04.85 Patentblatt 85/14**

(45) Bekanntmachung des Hinweises auf die Patenterteilung : **02.03.88 Patentblatt 88/09**

(84) Benannte Vertragsstaaten : **AT BE CH DE FR GB IT LI LU NL SE**

(56) Entgegenhaltungen :
**EP-A- 0 044 807**
**EP-A- 0 070 779**
**FR-A- 2 370 041**
**CHEMICAL ABSTRACTS SERVICE - REGISTRY HANDBOOK, Number Section 1965-1971, Registry Numbers 4600-00-4 through 12699-98-8 Amercian Chemical Society**
**JOURNAL OF THE CHEMICAL SOCIETY; Perkin Transactions I. 1978, Seiten 633-638 London, GB G.R. Brown et al.: "Sulphides Sulphoxides, and Sulphones derived from Salicyclic Acids"**

(73) Patentinhaber : **HOECHST AKTIENGESELLSCHAFT**
**Postfach 80 03 20**
**D-6230 Frankfurt am Main 80 (DE)**

(72) Erfinder : **Englert, Heinrich Christian, Dr.**
**Wielandstrasse 6**
**D-6238 Hofheim am Taunus (DE)**
Erfinder : **Lang, Hans-Jochen, Dr.**
**Rüdesheimer Strasse 7**
**D-6238 Hofheim am Taunus (DE)**
Erfinder : **Mania, Dieter, Dr.**
**Berliner Ring 5**
**D-6233 Kelkheim (Taunus) (DE)**
Erfinder : **Bickel, Martin, Dr.**
**Mittelstedter Weg 3**
**D-6380 Bad Homburg (DE)**

**EP 0 135 860 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 6-Sulfoxyphenolderivate der allgemeinen Formel I

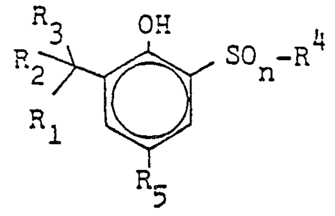

(I)

in welcher

$R^1$ für Wasserstoff, Alkyl mit 1 bis 4 C-Atomen, $OR^7$, wobei $R^7$ Wasserstoff oder Alkyl mit 1 bis 4 C-Atomen bedeutet, oder $NR^8R^9$, wobei $R^8$, $R^9$ gleich oder verschieden sind und Wasserstoff oder Alkyl mit 1 bis 4 C-Atomen bedeuten, steht ;

$R^2$, $R^3$ gleich oder verschieden sind und für $OR^{10}$, $NR^{10}R^{11}$ oder $SR^{10}$ stehen, wobei $R^{10}$ und $R^{11}$ gleich oder verschieden sind und für Alkyl mit 1 bis 8 C-Atomen stehen oder

$R^2$, $R^3$ gemeinsam eine Kette $—X—(CH_2)_m—X—$ bilden, wobei X für O, S oder $NR^{11}$ steht und m 2 bis 6 bedeutet, oder

$R^2$, $R^3$ gemeinsam für eine Carbonylfunktion =O oder eine Iminfunktion $=N\text{-}R^{11}$ stehen ;

$R^4$ für Alkyl mit 1 bis 4 C-Atomen, Cycloalkyl mit 3 bis 6 C-Atomen, Alkenyl mit 2 bis 4 C-Atomen und bis zu 2 Doppelbindungen und jeweils bis zu 3 Halogen-atomen oder für $NR^{12}R^{13}$ steht, wobei $R^{12}$ und $R^{13}$ gleich oder verschieden sind und Wasserstoff oder Alkyl mit 1 bis 4 C-Atomen bedeuten ;

$R^5$ für Alkyl mit 1 bis 10 C-Atomen oder Cycloalkyl mit 3 bis 8 C-Atomen und bis zu 8 Ringgliedern oder für Halogen steht ; und

n 1 oder 2 beträgt,

jedoch mit Ausnahme von 2-Hydroxy-5-methyl-3-methylsulfinylacetophenon, 2-Hydroxy-5-methyl-3-methylsulfonylacetophenon, 5-Chlor-3-methylsulfonylsalicylsäure, 3-Brom-5-methylsulfonylsalicylsäure und von 5-Brom-3-n-butyl-sulfonylsalicylsäure.

Bevorzugt sind Verbindungen der Formel I, in denen

$R^1$ für Wasserstoff oder OH steht ;

$R^2$, $R^3$ gemeinsam eine Carbonylfunktion oder eine Kette

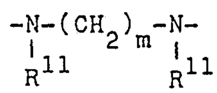

bilden, wobei m = 2 oder 3 ist und $R^{11}$ Methyl oder Ethyl bedeuten ;

$R^4$ Methyl oder $NH_2$ bedeutet ;

$R^5$ Alkyl mit 4 bis 8 C-Atomen bedeutet, und

n 1 oder 2 bedeutet.

Besonders bevorzugt sind Verbindungen der Formel I mit $R^1$ für Wasserstoff, $R^2$ und $R^3$ gemeinsam für die Carbonylfunktion, $R^4$ für Methyl, $R^5$ für Alkyl mit 4 bis 8 C-Atomen und n für 1 oder 2. Insbesondere haben sich solche Verbindungen bewährt, in denen $R^5$ 1,1-Dimethylethyl und n = 2 bedeuten ; ebenfalls eine solche Verbindung mit $R^5$ 1,1-Dimethylethyl und n = 1.

Weiterhin bevorzugt ist eine Verbindung der allgemeinen Formel I mit $R^1$ für Wasserstoff, $R^2$ und $R^3$ gemeinsam für die Carbonylfunktion, $R^4$ für Chlormethyl, $R^5$ für 1,1-Dimethylethyl und n für 2 ; des weiteren eine Verbindung mit $R^1$ für Wasserstoff, $R^2$ und $R^3$ gemeinsam für die Carbonylfunktion, $R^4$ für $NH_2$, $R^5$ für 1,1-Dimethylethyl und n = 2. Ebenfalls von besonderer Bedeutung ist eine Verbindung mit $R^1$ für Wasserstoff, $R^2$ und $R^3$ gemeinsam für

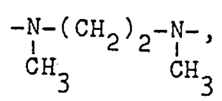

$R^4$ für Methyl,

$R^5$ für 1,1-Dimethylethyl und

n = 2.

Insbesondere sind Verbindungen nach Formel I bevorzugt, in denen $R^1$ für Wasserstoff oder OH steht ;

$R^2$, $R^3$ gemeinsam für die Carbonylfunktion =O stehen ;

$R^4$ $NR^{12}R^{13}$ bedeutet, wobei $R^{12}$ und $R^{13}$ gleich oder verschieden, sind, und Wasserstoff oder Alkyl

mit 1 bis 4 C-Atomen bedeuten ;

$R^5$ für Alkyl mit 3 bis 8 C-Atomen ; und

n für 2 steht.

Die Erfindung betrifft weiterhin ein Verfahren zum Herstellen einer Verbindung der allgemeinen Formel I nach Anspruch 1, welches dadurch gekennzeichnet ist, daß man

a) Verbindungen der Formel II

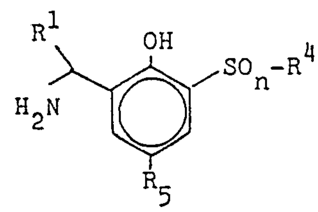

(II)

in der $R^1$ Wasserstoff oder Alkyl mit 1 bis 4 C-Atomen, $R^4$, $R^5$ und n die obengenannten Bedeutungen aufweisen, $R^4$ jedoch zusätzlich auch für eine Gruppierung —N = Z stehen kann, wobei Z die Bedeutung einer Schutzgruppe der Formel V zukommt,

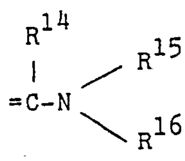

(V)

in der die Reste $R^{14}$ Wasserstoff oder Alkyl mit 1 bis 4 C-Atomen und $R^{15}$, $R^{16}$ Alkyl mit 1 bis 4 C-Atomen bedeuten, mit Formaldehyd oder einem formaldehyderzeugenden Reagenz umsetzt zu Verbindungen der Formel I und gegebenenfalls die Schutzgruppe Z hydrolytisch abspaltet ;

b) Verbindungen der Formel III

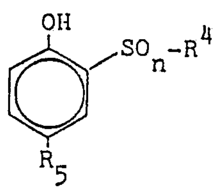

(III)

in welcher $R^4$, $R^5$ und n die obengenannten Bedeutungen haben, $R^4$ jedoch zusätzlich die unter Verfahrensvariante a) definierte Bedeutung —N = Z aufweisen kann, umsetzt mit einem Formylierungsreagenz zu Verbindungen der allgemeinen Formel I und gegebenenfalls die Schutzgruppe Z hydrolytisch abspaltet ;

c) Verbindungen der allgemeinen Formel IV

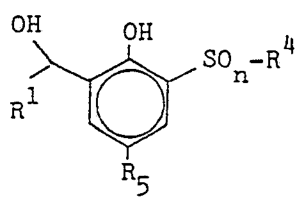

(IV)

in der $R^1$, $R^4$, $R^5$ und n die genannten Bedeutungen haben, $R^4$ jedoch zusätzlich die unter Verfahrensvariante a) definierte Bedeutung —N = Z aufweisen kann, oxidiert zu Verbindungen der allgemeinen Formel I und gegebenenfalls die Schutzgruppe Z hydrolytisch abspaltet ;

d) Verbindungen der Formel I, in der $R^1$ für Wasserstoff, $R^2$ und $R^3$ gemeinsam für die Carbonylfunktion stehen, $R^4$ und $R^5$ die obengenannten Bedeutungen haben, $R^4$ jedoch zusätzlich die unter Verfahrensvariante a) beschriebene Bedeutung —N = Z haben kann, oxidiert zu Verbindungen der allgemeinen Formel I und gegebenenfalls die Schutzgruppe Z hydrolytisch abspaltet und gegebenenfalls die erhaltenen Säuren in ihre Ester oder Amide der allgemeinen Formel I überführt ;

e) Verbindungen der allgemeinen Formel I, in der $R^1$ für Wasserstoff oder Alkyl mit 1 bis 4 C-Atomen steht ; $R^2$, $R^3$ gemeinsam eine Carbonylfunktion bilden ; $R^4$, $R^5$ und n die obengenannten Bedeutungen haben, mit Alkoholen $R^{10}OH$, Thioalkoholen $R^{10}SH$, Aminen $NHR^{10}R^{11}$ sowie mit Verbindungen $HX—(CH_2)_m—XH$, wobei $R^{10}$, $R^{11}$, X und m die obengenannten Bedeutungen haben, in die entsprechenden Acetale, Thioacetale, Aminale, oder Imine der allgemeinen Formel I überführt.

f) Verbindungen der allgemeinen Formel XVII

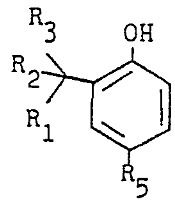

(XVII)

chlorsulfoniert und die entstandenen Sulfochloride

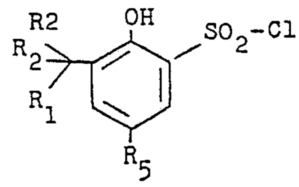

(XVIII)

entweder mit Aminen

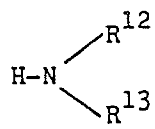

umsetzt zu den Verbindungen I oder einer Reduktion zu den Sulfinsäuren XIX

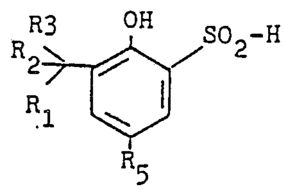

(XIX)

unterwirft und diese durch Alkylierung in die Verbindungen I überführt.

Die Erfindung betrifft des weiteren die Verwendung einer Verbindung der allgemeinen Formel I zur Behandhandlung und Prophylaxe von Schädigungen der Schleimhaut, des Magen-Darmtraktes und von Schädigungen der Leber, des Pankreas und des Gefäßsystems.

Verbindungen der allgemeinen Formel I sind neu. Sie zeigen, an Ratten appliziert, ausgeprägte cytoprotektive Eigenschaften, die für derartige Verbindungen nicht zu erwarten waren.

Falls Verbindungen der Formel I chirale C- und/oder S-Atome aufweisen, so sind sowohl R als auch S-konfigurierte Verbindungen Gegenstand der Erfindung. Die Verbindungen können dann in allen denkbaren diastereomeren Formen als Racemate, reine Enantiomere oder auch als Mischungen derselben vorliegen.

Bei der Umsetzung von Benzylaminen II zu den Verbindungen der allgemeinen Formel I gemäß Verfahrensvariante a) bedarf es im allgemeinen der Verwendung eines sauren Katalysators. Die Benzylamine können als solche oder in Form ihrer Säureadditionsalze eingesetzt und anstelle von Formaldehyd können auch sehr gut Formaldehyd-freisetzende Vorstufen, wie etwa Paraformaldehyd oder Urotropin (Hexamethylentetramin), verwendet werden. Saure Katalysatoren sind alle gängigen Mineralsäuren, aber auch organische Säuren sind vorteilhaft, wie etwa Alkansäuren, da diese direkt auch gleichzeitig als Lösemittel dienen können. Als besonders vorteilhaft hat sich die Verwendung der HCl-Additionssalze der Benzylamine II gezeigt, diese können in Trifluoressigsäure mit Urotropin glatt zu den Verbindungen I umgesetzt werden. Die Reaktionstemperatur kann in weiten Grenzen schwanken, vorteilhaft sind Temperaturen zwischen 40 und 120 °C. Im allgemeinen arbeitet man zweckmäßig bei Rückflußtemperatur des verwendeten Lösemittels. Insbesondere bei Verwendung von Urotropin kann gegen Ende der Reaktion der Zusatz von geringen Mengen wäßriger, starker Mineralsäure, wie etwa 4 N Salzsäure, von Vorteil sein, um die Ausbeute zu verbessern. Bei Verwendung handelsüblicher Trifluoressigsäure als Lösemittel ist dies jedoch meist nicht notwendig. Es ist dagegen unumgänglich, wenn Verbindungen I mit $R^4 = NH_2$ hergestellt werden, da dabei die oben erwähnte Schutzgruppe Z abgespalten wird.

Die Reaktionsgemische werden entweder durch Abdestillieren des Lösemittels oder durch Verdünnen mit einem Nichtlösemittel, wie etwa Wasser, aufgearbeitet. Die Produkte fallen dabei meist in kristalliner Form an und können durch Umkristallisation aus geeigneten gängigen Lösemitteln oder durch Chromatografie weiter gereinigt werden. In vielen Fällen ist eine zusätzliche Reinigung nicht notwendig.

Die Benzylamine II können leicht nach literaturbekannten Verfahren, beispielsweise durch Amidoalkylierung mit N-Hydroxymethylcarboxamiden der allgemeinen Formel VI von Phenolen III unter Säurekatalyse hergestellt werden :

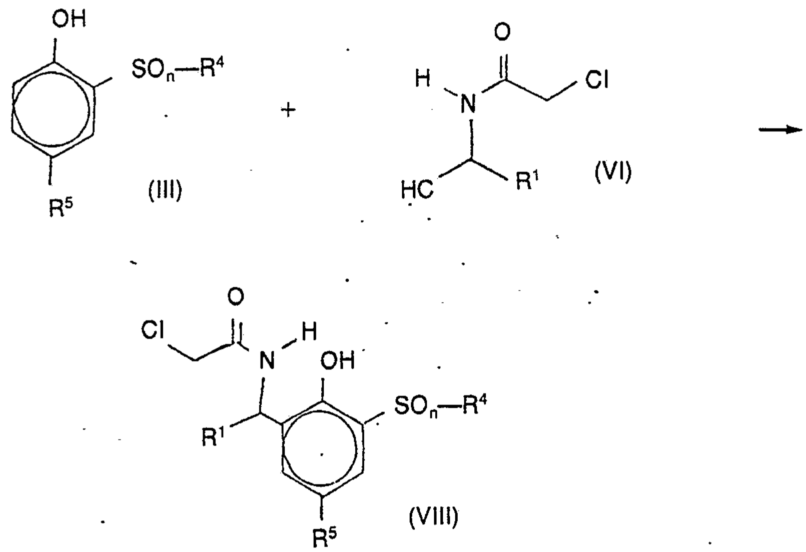

(III) (VI) (VIII)

Die dabei entstehenden Amide VIII lassen sich leicht durch saure Hydrolyse zu den Benzylaminen II umsetzen. Die Reste $R^4$, $R^5$ sowie n haben hierbei die obengenannten Bedeutungen, $R^1$ ist H oder $C_1$-$C_4$-Alkyl.

Die Phenole der allgemeinen Formel III können nach verschiedenen Verfahren hergestellt werden. Eine Methode besteht z. B. darin, daß man Thioether der allgemeinen Formel IX oxidiert,

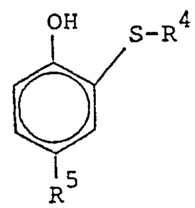

(IX)

wobei die Reste $R^4$ und $R^5$ sowie n die oben erwähnten Bedeutungen haben. Derartige Oxidationen sind literaturbekannt. Weiterhin ist bekannt, daß man durch Wahl der Reaktionsbedingungen entweder Sulfoxide (n = 1) oder Sulfone (n = 2) erhalten kann.

Die Thioether der allgemeinen Formel IX können in an sich bekannter Weise aus den Phenolen der Formel X, in der $R^5$ obige Bedeutung hat, hergestellt werden ; z. B. durch Einwirken eines Sulfoxides $R^4$—SO—$R^4$ in Gegenwart von Perchlorsäure und Phosphoroxychlorid oder durch Reaktion mit einem Sulfenylchlorid $R^4$—S—Cl in an sich bekannter Weise, wobei $R^4$ jeweils die oben erwähnte Bedeutung hat.

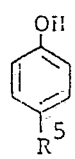

(X)

Die Phenole X können leicht durch Standardmethoden (vgl. Houben-Weyl, Methoden der org. Chem.- Phenole, Teil 2, S. 925 ff, G. Thieme Verlag, Stuttgart 1976) hergestellt werden.

Eine weitere Methode zur Herstellung von Phenolen der allgemeinen Formel III, in der $R^5$ und $R^4$ die obengenannten Bedeutungen haben und n = 2 ist, besteht in der Etherspaltung von Anisolen der allgemeinen Formel XI mit entsprechenden Bedeutungen von $R^5$, $R^4$ und n.

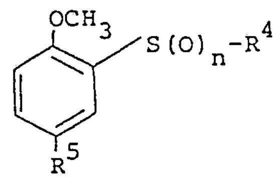

(XI)

Sie wird in an sich bekannter Weise durch Einwirkung von Mineralsäuren wie Jodwasserstoffsäure oder von Lewissäuren wie Aluminiumchlorid oder Bortribromid in inerten Lösungsmitteln, wie z. B. Methylenchlorid oder Chloroform, vorgenommen. Auch die gängige Spaltung durch Pyridiniumhydrochlorid bei Temperaturen oberhalb 180 °C kann erfolgreich durchgeführt werden.

Verbindungen der allgemeinen Formel XI mit n = 2 werden durch eine Sequenz an sich bekannter Standardmethoden aus den Anisolen XII hergestellt :

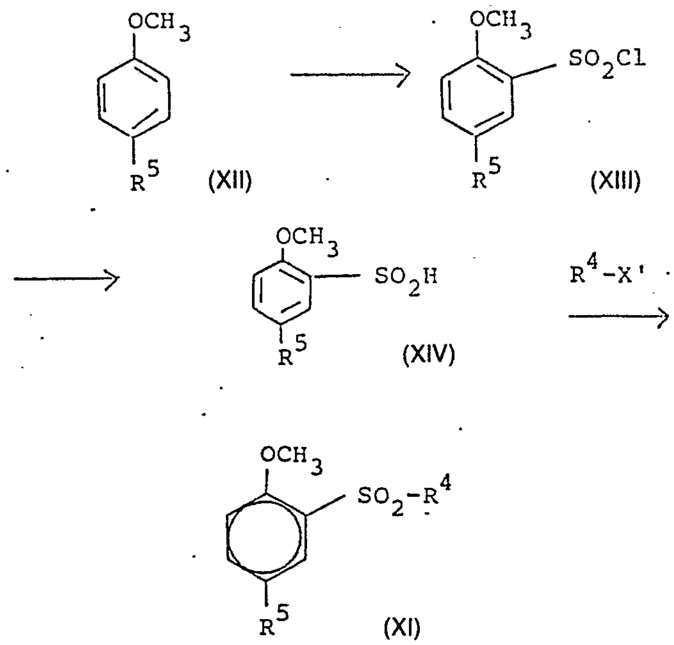


(XII) (XIII) (XIV) (XI)

Durch Einwirkung von Chlorschwefelsäure auf Anisole XII erhält man in an sich bekannter Weise Sulfochloride XIII. Vorteilhaft wird diese Reaktion in einem inerten Lösemittel wie Chloroform oder Methylenchlorid durchgeführt. Wenn $R^5$ in XII für höheres Alkyl steht, ist ein Temperaturbereich von 0-20 °C einzuhalten und ein an sich üblicher, größerer Überschuß von mehr als 3 Äquivalenten Chlorsulfonsäure zu vermeiden.

Die Reduktion zu den Sulfinsäuren XIV kann nach den verschiedensten Verfahren (vgl. Houben-Weyl, Methoden der org. Chemie, Band X, S. 563 ff., G. Thieme Verlag, Stuttgart, 1955) durchgeführt werden. Als einfache und effiziente Methode erwies sich die Reduktion mit Natriumsulfit in Gegenwart von Natriumhydroxid in wäßrig-acetonischen Lösungen.

Die Alkylierung von Sulfinsäuren zu Sulfonen ist eine an sich bekannte Reaktion, wobei vorzugsweise unter Basenkatalyse gearbeitet wird. Im Falle der Sulfinsäuren XIV erweisen sich Alkyljodide als besonders geeignete Alkyllierungsmittel $R^4$—X', wobei $R^4$ die obengenannte Bedeutung hat und X' für Halogen, vorzugsweise für Jod steht. Als Basen werden vorteilhaft organische Basen, wie Triethylamin in acetonischer Lösung, aber auch anorganische Basen wie LiOH, NaOH, KOH usw. in wäßriger, oder wäßrig-acetonischer Lösung eingesetzt.

Steht $R^4$ für einen α-Halogenalkylrest, so können eine Reihe Varianten dieser Alkylierungsreaktion vorteilhaft zur Anwendung kommen, wie z. B. die Umsetzung von Sulfinsäuren mit α-Dihalogencarbonsäuren in Gegenwart von $K_2CO_3$, wobei die intermediär gebildete β-Sulfonyl-α-halogencarbonsäure in an sich bekannter Weise $CO_2$ abspaltet unter Bildung des α-Halogenmethylsulfonylrestes.

Die Anisole XII werden durch Standardmethoden (Organikum, VEB Deutscher Verlag der Wissenschaften, Berlin 1971, S. 222) aus den Phenolen X hergestellt.

Phenole der allgemeinen Formel III, bei denen $R^4$ in der Bedeutung $NR^{12}R^{13}$ oder —N = Z steht, wobei $R^{12}$, $R^{13}$ die obengenannten Bedeutungen haben, können leicht und nach Standardmethoden hergestellt werden. Durch Einwirken von Aminen $NHR^{12}R^{13}$ auf die Sulfochloride XIII erhält man die Sulfonamide XV

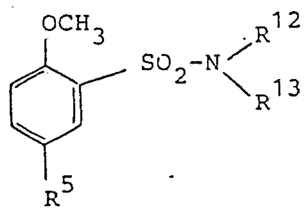

(XV)

Für den Fall, daß $R^{12}$, $R^{13}$ die Bedeutung von Wasserstoff haben, können die Sulfonamide XV gegebenenfalls analog wie in der Deutschen Offenlegungsschrift 2 658 766 oder in der DE-OS 24 61 601 beschrieben durch Einleitung von substituierten Formamiden, z. B. von Dimethylformamid in Gegenwart eines die Wasserabspaltung begünstigenden Reagenz wie Thionylchlorid oder Phosphoroxychlorid zu den geschützten Sulfonamiden XVI umgesetzt werden. Anstelle der Formamide können auch deren Acetale eingesetzt werden, wie z. B. Dimethylformamiddimethylacetal, in diesem Fall läuft die Reaktion dann meist ganz ohne Zusatz eines Kondensationsmittels ab.

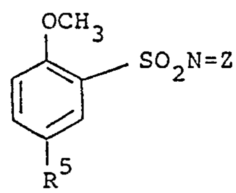

(XVI)

Durch Etherspaltung, wie für die Anisole XI beschrieben, können auch die Anisole XV und XVI zu den entsprechenden Phenolen III umgesetzt werden.

Werden Verbindungen I, insbesondere solche, bei denen $R^2$, $R^3$ gemeinsam für eine Carbonylgruppe stehen und $R^1$ Wasserstoff bedeutet, nach Verfahrensvariante b) aus den Phenolen III hergestellt, so geschieht dies in an sich bekannter Weise durch alle für Phenole geeigneten Formylierungsreaktionen, beispielsweise solche, die in « Advanced Organic Chemistry », Jerry March, Mc-Graw Hill, Series in Advanced Chemistry, Mc-Graw Hill Koga Kusha Ltd, 1977, Seite 1167 beschrieben sind.

Als besonders vorteilhaft hat sich die Anwendung der Duff-Reaktion (siehe Chem. Rev. 38, 230 ff. (1946)) erwiesen, beispielsweise durch Verwendung von Urotropin in einem sauren organischen Lösemittel wie etwa Alkansäuren. Ganz besonders vorteilhaft ist hier die Verwendung von Trifluoressigsäure. Für Durchführung und Aufarbeiten der Reaktion sind die selben Bedingungen wie unter Verfahrensvariante a) beschrieben anwendbar und vorteilhaft.

Werden Verbindungen I aus den Verbindungen IV durch Oxidation gemäß Verfahrensvariante c) hergestellt, so kann dies im Prinzip mit allen gängigen Oxidationsmitteln durchgeführt werden. Beispielsweise seien $KMnO_4$, Brom, $MnO_2$, $RhO_4$, $CrO_3$, $Na_2Cr_2O_7$, Silbersalze wie $Ag_2CO_3$, $Ag_2O$, Pyridiniumchlorochromat und viele mehr, wie sie beispielsweise in « Advanced Organic Chemistry », Jerry March, Mc-Graw-Hill Kogakusha Ltd., 1977, Seite 1082-1088), für derartige Reaktionen beschrieben sind. Als besonders vorteilhaft hat sich beispielsweise die Verwendung von $Na_2Cr_2O_7$ unter Zusatz von Mineralsäure wie Schwefelsäure in einem Zweiphasensystem aus Wasser und einem organischen Lösemittel wie etwa Diethylether erwiesen. Steht $R_1$ in den Verbindungen IV für Wasserstoff, so ist auch die Verwendung von Pyridiniumchlorochromat in chlorierten Kohlenwasserstoffen, beispielsweise Methylenchlorid, von Bedeutung, besonders dann, wenn Verbindungen I mit $R_1$ = H hergestellt werden sollen.

Die Verbindungen IV können auf verschiedenen Wegen hergestellt werden. Eine Möglichkeit besteht beispielsweise darin, daß man die Phenole III einer Hydroxymethylierung unterwirft. Man verfährt dabei im Allgemeinen so, daß man deren Alkalimetallsalze, beispielsweise die Natriumsalze, in wäßriger Lösung mit Formaldehyd oder einem formaldehydgenerierenden Reagens, wie etwa Paraformaldehyd, zur Reaktion bringt. Man erhält so in an sich bekannter Weise Verbindungen IV mit $R^1$ = H.

Verbindungen mit $R^1$ in der Bedeutung niedrig Alkyl können weiterhin durch Reaktion von Verbindungen der allgemeinen Formel I mit $R_1$ = H ; $R^2$, $R^3$ = Carbonyl mit Grignard-Reagentien der allgemeinen Formel $R^1$—Mg—Hal, wobei $R^1$ für Alkyl mit 1 bis 4 C-Atomen steht und Hal hier für Chlor oder Brom steht, in an sich bekannter Weise erhalten werden. Durch Zusatz von N,N,N',N'-Tetramethylethylendiamin läuft die Reaktion glatt ab.

Bei der Herstellung der Verbindungen I, in denen $R^2$, $R^3$ gemeinsam eine Carbonylfunktion bilden und $R^1$ für OH, $OR^7$ oder $NR^8R^9$ steht, kann man auch so vorgehen, daß man zunächst gemäß Verfahrensvariante d) Verbindungen I mit $R^2$, $R^3$ = Carbonyl und $R^1$ = Wasserstoff oxidiert, wobei prinzipiell die selben Oxidationsmittel anwendbar sind, wie die unter Verfahrensvariante c) aufgeführten, insbesondere solche, die in Kombination mit einem wäßrigen Lösemittel anwendbar sind. Besonders vorteilhaft erwies sich dabei die Verwendung von Permanganaten, beispielsweise $KMnO_4$ in alkalisch-

wäßriger Lösung wie etwa in Natronlauge. Die Reaktion läuft meist schon bei Zimmertemperatur ab, zur Erzielung guter Ausbeuten bedarf es jedoch höherer Temperaturen. Vorteilhaft läuft die Reaktion zwischen 40 und 80 °C ab. Überschüssiges Oxidationsmittel wird meist durch ein mildes Reduktionsmittel, wie etwa $Na_2SO_3$, gegen Ende der Reaktion zerstört. Durch Ansäuern der Reaktionslösungen mit Mineralsäure, wie etwa 2 N HCl, gewinnt man die Reaktionsprodukte in reiner kristalliner Form. Die erhaltenen Carbonsäuren der allgemeinen Formel I mit $R^1$ in der Bedeutung OH können dann gegebenenfalls durch Standardmethoden in ihre Ester überführt werden, beispielsweise durch den säurekatalysierten Umsatz mit Alkoholen $R^7OH$, etwa dadurch, daß man die Säure I in $R^7OH$ als Lösemittel, wobei $R^7$ die obengenannte Bedeutung hat, unter Zusatz von Schwefelsäure zum Rückfluß erhitzt. Aber auch andere bekannte Veresterungsmethoden können benutzt werden, etwa die Methode der gemischten Anhydride oder die Säurehalogenidmethode. Entsprechendes gilt, wenn aus den Verbindungen I mit $R_1$ in der Bedeutung OH die entsprechenden Carbonsäureamide I mit $R^1$ in der Bedeutung $NR^8R^9$ herzustellen sind. Auch hierbei können alle Standardmethoden zur Herstellung von Carbonsäureamiden aus Carbonsäuren angewendet werden.

Verbindungen I, bei denen $R_1$ in der Bedeutung Wasserstoff oder Alkyl mit 1 bis 4 C-Atomen steht und bei denen $R_2$, $R_3$ gemeinsam für die Carbonylgruppe stehen, lassen sich gemäß Verfahrensvariante e) nach Standardmethoden in solche Verbindungen I überführen, bei denen $R^2$, $R^3$ für $OR^{10}$, $SR^{10}$, $NR^{10}R^{11}$ steht und $R^{10}$, $R^{11}$ die obengenannten Bedeutungen haben, oder bei denen $R^2$, $R^3$ gemeinsam eine $X-(CH_2)_m-X$-Kette bilden mit X und m in den obengenannten Bedeutungen oder bei denen $R^2$, $R^3$ gemeinsam eine Iminfunktion $= N-R^{11}$ bilden. Es handelt sich hierbei um Verfahren zur Acetalisierung, Thioacetalisierung, Aminalbildung und Schiffbasenherstellung aus Carbonylverbindungen. Vorteilhaft bedient man sich bei der Acetalisierung und der Thioacetalisierung der durch Lewissäuren katalysierten Reaktion der Carbonylverbindungen I mit Alkoholen $HOR^{10}$ oder Thioalkoholen $HSR^{10}$ in einem den Reaktionspartnern gegenüber inerten Lösemittel, wie etwa chlorierte Kohlenwasserstoffe, beispielsweise Methylenchlorid in Gegenwart wasserentziehender Mittel wie etwa $MgSO_4$, Molsieb oder $TiCl_4$. Insbesondere bei der Acetalisierung kommen auch Mineralsäuren wie, $H_2SO_4$, als Katalysator in Betracht.

Primäre Amine $H-NR^{10}R^{11}$ ergeben im Allgemeinen ohne Zusatz von Katalysatoren oder wasserentziehenden Mitteln Imine der allgemeine Formel I mit $R^2$, $R^3$ in der Bedeutung von $= N-R^{11}$, während sekundäre Amine $H-NR^{10}R^{11}$, insbesondere aber Diamine der allgemeinen Formel $H-NR^{11}-(CH_2)_m-NR^{11}-H$ sehr leicht Aminale bilden. Meist genügt schon einfaches Mischen der Ausgangskomponenten in einem inerten Lösemittel, wie etwa Methanol oder $CH_2Cl_2$. Die gewünschten Verbindungen können dann durch einfaches Entfernen des Lösemittels, vorteilhaft durch Eindampfen, isoliert werden.

Bei allen Verfahrensvarianten können auch Ausgangsmaterialien der allgemeinen Formel II, III, IV, V, VI, bei denen $R^4$ in der Bedeutung

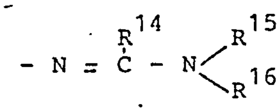

steht und $R^{14}$ bis $R^{16}$ obengenannten Bedeutungen haben, Anwendung finden für die Herstellung von Verbindungen I mit $R^4$ in der Bedeutung $NH_2$.

Der Gruppierung

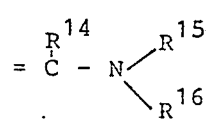

kommt dann die Bedeutung einer Schutzgruppe für die $NH_2$-Funktion zu. Bei ihrer Verwendung ist es dann notwendig, im Anschluß an die jeweilige Verfahrensvariante diese Schutzgruppe hydrolytisch abzuspalten. Dies wird in an sich bekannter Weise vorgenommen, vorteilhaft dadurch, daß man die zu hydrolysierende Verbindung in wäßriger Alkylihydroxidlösung, beispielsweise 2 N Natronlauge, auflöst und anschließend durch Ansäuern der Lösung, beispielsweise mit 2 N Salzsäure, die von der Schutzgruppe befreiten Verbindungen I in kristalliner Form erhält.

Wird bei der Verfahrensvariante f so vorgegangen, daß man zunächst Phenole der allgemeinen Formel XVII zu den Sulfochloriden XVIII umsetzt, so kann dies beispielsweise dadurch erzielt werden, daß man zunächst ein Sulfonierungsmittel wie etwa Schwefelsäure oder Chlorsulfonsäure auf die Phenole XVII einwirken läßt und nachfolgend ein Chlorierungsmittel zugibt, wie etwa Sulfurylchlorid, Phosphoroxychlorid oder Chlorsulfonsäure. Vorzugsweise kommt ein Verfahren zur Anwendung, bei dem man 2 oder mehr Äquivalente Chlorsulfonsäure auf die Phenole XVII einwirken läßt. Man verwendet die für Chlorsulfonierung üblichen Lösemittel wie etwa Chlorkohlenwasserstoffe (z. B. Methylenchlorid) oder man arbeitet ganz ohne Lösemittel, was beispielsweise für Phenole XVII besonders geeignet ist, bei

denen $R_2$, $R_3$ gemeinsam die Carbonylfunktion bedeuten. Die Reaktionsprodukte XVIII werden durch Zugabe eines Nichtlösemittels wie etwa Wasser isoliert und anschließend entweder nach Standardmethoden mit Aminen

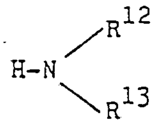

umgesetzt oder analog wie unter Verfahrensvariante a) für die Umsetzung von Verbindungen XIII über XIV zu XI beschrieben, zu den Sulfinsäuren XIX umgesetzt, welche durch Alkylierung mit $R^4$-X', wobei $R^4$ und X' die obengenannten Bedeutungen haben, in Verbindungen I umgewandelt werden.

Soweit nicht ausdrücklich anders erwähnt, bedeuten in der vorliegenden Beschreibung die Begriffe Alkyl Alkylgruppen mit 1 bis 8, vorzugsweise 1 bis 4, insbesondere 1 bis 2 C-Atomen, und Halogen Fluor, Chlor, Brom und Jod, bevorzugt Chlor, Brom, Jod, insbesondere Chlor und Brom.

Erfindungsgemäß können außer den in den Ausführungsbeispielen beschriebenen Verbindungen auch die in der folgenden Tabelle zusammengestellten Verbindungen der allgemeinen Formel I erhalten werden :

| $R^1$ | $R^2$ | $R^3$ | $R^4$ | $R^5$ | n |
|---|---|---|---|---|---|
| H | =O | | $NH_2$ | Cl | 2 |
| H | =O | | $NH_2$ | Br | 2 |
| H | =O | | $NH_2$ | F | 2 |
| H | =O | | $NH_2$ | J | 2 |
| H | =O | | $CF_3$ | tert. Bu | 2 |
| H | =O | | $CF_3$ | tert. Bu | 1 |
| H | =O | | $CH_2$-Cl | tert. Bu | 1 |
| H | $OCH_3$ | $OCH_3$ | $CH_3$ | tert. Bu | 2 |
| H | $OCH_3$ | $OCH_3$ | $CH_3$ | tert. Bu | 1 |
| H | $SCH_3$ | $SCH_3$ | $CH_3$ | tert. Bu | 2 |
| H | $-O-(CH_2)_2-O$ | | $CH_3$ | tert. Bu | 2 |
| H | $-S-(CH_2)_2-S$ | | $CH_3$ | tert. Bu | 2 |
| H | $-O-(CH_2)_2-S$ | | $CH_3$ | tert. Bu | 2 |

| $R^1$ | $R^2$ | $R^3$ $R^4$ | $R^5$ | n |
|---|---|---|---|---|
| H | =O | $NH_2$ | $C_3H_7$ | 2 |
| H | =O | $NH_2$ | iso-$C_3H_7$ | 2 |
| H | =O | $NH_2$ | sec. $C_4H_9$ | 2 |
| H | =O | $NH_2$ | tert.-Amyl | 2 |
| H | =O | $NH_2$ | cyclo-$C_5H_9$ | 2 |
| H | =O | $NH_2$ | cyclo-$C_6H_{11}$ | 2 |
| H | =O | $NH_2$ | n-Bu | 2 |
| $CH_3$ | =O | $NH_2$ | tert.-Bu | 2 |
| OH | =O | $NH_2$ | tert.-Bu | 2 |
| $OCH_3$ | =O | $NH_2$ | tert.-Bu | 2 |
| $NH_2$ | =O | $NH_2$ | tert.-Bu | 2 |
| $HN-CH_3$ | =O | $NH_2$ | tert.-Bu | 2 |
| $N(CH_3)_2$ | =O | $NH_2$ | tert.-Bu | 2 |
| H | =O | $NHCH_3$ | tert.-Bu | 2 |
| H | =O | $N(CH_3)_2$ | tert.-Bu | 2 |

| $R^1$ | $R^2$ | $R^3$ | $R^4$ | $R^5$ | n |
|---|---|---|---|---|---|
| H | $-O-(CH_2)_2-O-$ | | $NH_2$ | tert.-Bu | 2 |
| H | $-S-(CH_2)_2-O-$ | | $NH_2$ | tert.-Bu | 2 |
| H | $-N(CH_3)-(CH_2)_2-N(CH_3)-$ | | $NH_2$ | tert.-Bu | 2 |
| H | $-O-(CH_2)_3-O-$ | | $NH_2$ | tert.-Bu | 2 |
| H | $-S-(CH_2)_3-S-$ | | $NH_2$ | tert.-Bu | 2 |
| H | $OCH_3$ | $OCH_3$ | $NH_2$ | tert.-Bu | 2 |
| H | $SCH_3$ | $SCH_3$ | $NH_2$ | tert.-Bu | 2 |
| H | $=N-CH_3$ | | $NH_2$ | tert.-Bu | 2 |
| H | $=N-C_2H_5$ | | $NH_2$ | tert.-Bu | 2 |
| H | $=N-CH_3$ | | $CH_3$ | tert.-Bu | 2 |
| H. | $=N-CH_3$ | | $CH_3$ | tert.-Bu | 1 |

Die Verbindungen der allgemeinen Formel I sind von besonderem pharmakologischen und medizinischen Interesse, da sie cytoprotektiv wirksam sind. Dies bedeutet, daß sie Schädigungen von menschlichen oder tierischen Zellen verhindern, die durch exogene oder endogene Noxen oder durch altersbedingte degenerative Prozesse hervorgerufen werden. Eine derartige resistenzsteigernde Wirkung der Substanzen I auf Oberflächenepithelien kann beispielsweise zur Vorbeugung und Heilung von Schleimhautschäden des Magen-Darmkanals, wie etwa des Ulcus duodeni eventriculi, sowie der Gastritis benutzt werden. Aber auch an anderen Organen können die Verbindungen cytoprotektiv wirksam sein, beispielsweise bei akuten chronischen Schädigungen der Leber, des Pankreas und von Gefäßen.

Zur Definition des Begriffes « Cytoprotektion » sei auf C. Johansson und S. Bergström in J. of Gastroenterol. 1982, Suppl. nr. 77, S. 21 ff. hingewiesen.

Die erfindungsgemäßen Verbindungen der Formel I sind Cytoprotektiva, die als Pharmazeutika in der Human- und Veterinärmedizin, eingesetzt werden können. Sie werden in Dosierungen von 0,01 bis 30 mg/kg · Tag, vorzugsweise von 0,1 bis 10 mg/kg · Tag, insbesondere von 0,3 bis 3 mg/kg · Tag in Kapseln, Dragees, Tabletten oder Lösung mit verschiedenen Zusätzen enteral, z. B. oral mit Sonde oder dgl. oder parenteral (Injektion in das Gefäßsystem, z. B. intravenös oder auch Injektionen in die Muskulatur oder auch transdermal) verabfolgt. Sie eignen sich sowohl zur Behandlung als auch zur Prophylaxe von Schleimhautschäden im Bereich des Magen-Darmkanals, von Schäden an Leber, Pankreas und an Gefäßen.

Die Verbindungen können allein oder in Kombination mit anderen pharmazeutischen Wirkstoffen angewendet werden, insbesondere sind solche Pharmazeutika zu nennen, die neben ihrer therapeutischen Wirksamkeit unvermeidbare schädigende Wirkungen entfalten, beispielsweise nichtsteroidale Antiphlogistika, wie Aspirin oder Indometacin, oder etwa Corticoide, wie Cortison, aber auch Zytostatika, wie etwa 5-Fluoruracil oder Cyclophosphamid, seien genannt. Halogen bedeutet, wenn nicht ausdrücklich anders erwähnt, Cl, Br oder J, vorzugsweise Cl oder Br.

Beispiel 1

2-Formyl-4-(1,1-dimethylethyl)-6-methylsulfonylphenol

14,65 g (0,05 Mol) 2-Aminomethyl-4-(1,1-dimethylethyl)-6-methylsulfonylphenolhydrochlorid werden in 100 ml Trifluoressigsäure gelöst, mit 9 g (0,065 Mol) Urotropin (Hexamethylentetramin) versetzt und 2,5 h am Rückfluß gekocht. Anschließend gibt man 50 ml 4 N Salzsäure zu, kocht erneut für 10 min am Rückfluß, gießt die Reaktionsmischung auf Eiswasser und saugt ab. Hellgelbe.

Kristalle vom Schmp. : 134-136 °C.

Herstellung der Ausgangsverbindung 2-Aminomethyl-4-(1,1-dimethylethyl)-6-methylsulfonylphenolhydrochlorid

a) 3-(1,1-Dimethylethyl)-6-methylbenzolsulfonsäurechlorid

12,3 g (0,075 Mol) 4-(1,1-Dimethylethyl) phenol in 30 ml Methylenchlorid tropft man unter Eiskühlung zu 16,5 ml Chlorsulfonsäure gelöst in 20 ml Methylenchlorid. Man rührt das Gemisch 40 Minuten und gießt auf Eiswasser. Die organische Phase wird abgetrennt, mit Wasser gewaschen, mit $MgSO_4$ getrocknet und eingeengt. Die Umkristallisation erfolgt aus Toluol-Petrolether.

Kristalle vom Schmp. : 75-77 °C.

b) 3-(1,1-Dimethylethyl)-6-methoxybenzolsulfinsäure

10,7 g (0,04 Mol) 3-(1,1-Dimethylethyl)-6-methoxybenzolsulfonsäurechlorid werden in eine Lösung von 15 g $Na_2SO_3$ und 4 g NaOH in 100 ml Wasser eingetragen. Nach Zusatz von wenig Aceton wird die Mischung 30 Minuten auf dem Dampfbad erhitzt, filtriert und mit konz. Salzsäure auf pH 2-3 gebracht.
Farblose Kristalle vom Schmp. : 105-107 °C.

c) 4-(1,1-Dimethylethyl)-2-methylsulfonylanisol

17,4 g (0,076 Mol) 3-(1,1-Dimethylethyl)-6-methoxybenzolsulfinsäure werden in 120 ml Aceton suspendiert, mit 13,3 ml (0,095 Mol) Triethylamin sowie mit 8,5 ml (0,12 Mol) Jodmethan versetzt. Die Mischung wird 2 Stunden bei Raumtemperatur gerührt und anschließend auf Eiswasser gegossen. Der ausgefallene Niederschlag wird abgesaugt und aus n-Butanol umkristallisiert.
Weiße Kristalle vom Schmp. : 111-112 °C.

d) 4(1,1-Dimethylethyl)-2-methylsulfonylphenol

23,1 g (0,095 Mol) 4-(1,1-Dimethylethyl)-2-methylsulfonylanisol werden mit 60 g Pyridiniumhydrochlorid vermischt und 2 Stunden auf 210-220 °C erhitzt. Die erkaltete Masse wird in Wasser suspendiert, der sich abscheidende Feststoff abgesaugt und mit Petrolether ausgekocht. Man engt bis zur beginnenden Kristallisation ein.
Weiße Nadeln vom Schmp. : 103-104 °C.

e) 2-Chlor-N-[5-(1,1-dimethylethyl)-2-hydroxy-3-methylsulfonylbenzyl] acetamid

13,5 g (0,06 Mol) 4-(1,1-Dimethylethyl)-2-methylsulfonylphenol werden in 100 ml konz. Schwefelsäure gelöst. Man addiert 6,63 g (0,054 Mol) 2-Chlor-N-hydroxymethylacetamid und rührt 10 Minuten bei Raumtemperatur. Man gießt auf Eiswasser und kristallisiert das abgesaugte Rohprodukt aus Toluol um.
Farblose Kristalle vom Schmp. : 134-135 °C.

f) 2-Aminomethyl-4-(1,1-dimethylethyl)-6-methylsulfonylphenolhydrochlorid

6,3 g (0,028 Mol) 2-Chlor-N-[5-(1,1-dimethylethyl)-2-hydroxy-3-methylsulfonylbenzyl] acetamid werden in einer Mischung aus 20 ml konz. Salzsäure und 40 ml Ethanol 16 Stunden unter Rückfluß gekocht. Das ausgefallene Produkt wird abgesaugt und aus Ethanol umkristallisiert.
Farblose Nadeln vom Schmp. : 242-243 °C (Zers.)

## Beispiel 2

2-Formyl-4-(1,1-dimethylethyl)-6-Chlormethylsulfonylphenol

0,82 g (0,002 5 Mol) 2-Aminomethyl-4-(1,1-dimethylethyl)-6-chlormethylsulfonylphenolhydrochlorid werden analog Beispiel 1 mit Urotropin zum 2-Formyl-4-(1,1-dimethylethyl)-6-chlormethylsulfonylphenol umgesetzt.
Fahlgelbe Kristalle vom Schmp. : 128-129 °C.

Herstellung der Ausgangsverbindung 2-Aminomethyl-4-(1,1-dimethylethyl)-6-chlormethylsulfonylphenol-hydrochlorid

a) 2-Chlormethylsulfonyl-4-(1,1-dimethylethyl) anisol

22,8 g (0,1 Mol) 3-(1,1-Dimethylethyl-2-methoxybenzolsulfinsäure werden mit 23 g $Na_2CO_3$ und 15 g (0,117 Mol) Dichloressigsäure in 150 ml $H_2O$ gelöst. Die Lösung wird bei einer Badtemperatur von 160 °C langsam zur Trockne eingedampft. Man nimmt erneut mit $H_2O$ auf, neutralisiert mit wenig verdünnter Salzsäure und schüttelt mehrfach mit Ethylacetat aus. Nach Entfernen des Lösungsmittels fällt das Produkt kristallin an.
Schmp. : 114-116 °C.

b) 2-Chlormethylsulfonyl-4-(1,1-dimethylethyl) phenol

13,4 g (0,049 Mol) 2-Chlormethylsulfonyl-4-(1,1-dimethylethyl) anisol werden analog Beispiel 1 d) zum 2-Chlormethylsulfonyl-4-(1,1-dimethylethyl) phenol gespalten.
Schmp. : 94-95 °C.

c) 2-Chlor-N-[3-chlormethylsulfonyl-5-(1,1-dimethylethyl-2-hydroxybenzyl] acetamid

Diese Verbindung wird analog Beispiel 1 e) hergestellt, das Rohprodukt aus Toluol/Petrolether umkristallisiert.
Schmp. : 139-140 °C.

d) 2-Aminomethyl-4-(1,1-dimethylethyl)-6-chlormethylsulfonylphenolhydrochlorid

Diese Verbindung wird analog Beispiel 1 f) hergestellt. Umkristallisation aus Methanol/Ether liefert weiße Kirstalle vom Schmp. : 218-219 °C (Zers.)

Beispiel 3

2-Carboxy-4-(1,1-dimethylethyl)-6-methylsulfonylphenol

2,56 g (0,01 Mol) 2-Formyl-4-(1,1-dimethylethyl)-6-methylsulfonylphenol werden in eine Lösung von 1,58 g (0,01 Mol) Kaliumpermanganat in 50 ml 2 N Natronlauge eingetragen und 30 Minuten auf dem Dampfbad erhitzt. Anschließend wird die erkaltete Lösung mit 2 N Salzsäure auf pH 2 gebracht und mit konzentrierter $Na_2SO_3$-Lösung entfärbt. Man extrahiert 3 mal mit Essigester, engt im Vakuum ein und kristallisiert den Rückstand aus Acetonitril um.
Weiße Kristalle vom Schmp. : 226-227 °C.

Beispiel 4

2-Formyl-4-(1,1-dimethylpropyl)-6-methylsulfonylphenol

3,1 g (0,01 Mol) 2-Aminomethyl-4-(1,1-dimethylpropyl)-6-methylsulfonylphenolhydrochlorid werden analog Beispiel 1 mit Urotropin umgesetzt.
Kristalle vom Schmp. : 92-94 °C.

Herstellung des Ausgangsmaterials 2-Aminomethyl-4-(1,1-dimethylpropyl)-6-methylsulfonylphenolhydrochorid

Diese Verbindung wird analog der Reaktionsfolge 1 a) bis 1 f) hergestellt.
Schmp. : 187-188 °C (Zers.)

Beispiel 5

2-Formyl-4-brom-6-methylsulfonylphenol

3,16 g (0,01 Mol) 2-Aminomethyl-4-brom-6-methylsulfonylphenolhydrochlorid werden analog Beispiel 1 zum 2-Formyl-4-brom-6-methylsulfonylphenol umgesetzt.
Schmp. : 165-166 °C.

Herstellung der Ausgangsverbindung 2-Aminomethyl-4-brom-6-methylsulfonylphenolhydrochlorid

Diese Substanz wird analog der unter Beispiel 1 a) bis 1 f) beschriebenen Reaktionsfolge hergestellt.
Schmp. : 228-229 °C.

Beispiel 6

2-Formyl-4-(1,1-dimethylethyl)-6-ethylsulfonylphenol

0,615 g 2-Aminomethyl-4-(1,1-dimethylethyl)-6-ethylsulfonylphenolhydrochlorid werden analog Beispiel 1 mit 0,36 Urotropin in 8 ml Trifluoressigsäure zum 2-Formyl-4-(1,1-dimethylethyl)-6-ethylsulfonylphenol umgesetzt.
Schmp. : 92-93 °C.
Die Ausgangsverbindung 2-Aminomethyl-4-(1,1-dimethylethyl)-6-ethylsulfonylphenolhydrochlorid wird analog Beispiel 1 a bis 1 f hergestellt, jedoch wird im Verfahrensschritt 1 c anstelle von Methyljodid Ethyljodid eingesetzt.
Schmp. : 203-204 °C (Zers.)

Beispiel 7

2-Formyl-4-isopropyl-6-methylsulfonylphenol

0,7 g (0,002 5 Mol) 2-Aminomethyl-4-isopropyl-6-methylsulfonylphenolhydrochlorid werden analog Beispiel 1 mit 0,45 g Urotropin in 8 ml Trifluoressigsäure zum 2-Formyl-4-isopropyl-6-methylsulfonylphenol umgesetzt.
Schmp. : 114-116 °C.
Das Ausgangsmaterial 2-Aminomethyl-4-isopropyl-6-methylsulfonylphenolhydrochlorid wird analog Beispiel 1 a bis 1 f hergestellt, jedoch wird anstelle von 4-(1,1-dimethylethyl) anisol 4-Isopropylanisol in Stufe 1 a eingesetzt.
Schmp. : 246-247 °C.

Beispiel 8

2-Methoxycarbonyl-4-(1,1-dimethylethyl)-6-methylsulfonylphenol

1,0 g (0,003 Mol) des in Beispiel 3 hergestellten 2-Carboxy-4-(1,1-dimethylethyl)-6-methylsulfonylphenols werden in 6 ml Methanol gelöst, mit 0,1 ml konz. Schwefelsäure 2 Tage am Rückfluß erhitzt. Man gießt auf Eiswasser, extrahiert mit Ethylacetat, zieht das Lösemittel im Vakuum ab und unterwirft den Rückstand der Chromatographie an Kieselgel mit Ethylacetat/Toluol 4 : 1 als Elutionsmittel.
Weiße Kristalle vom Schmp. : 107-108 °C.

Beispiel 9

2-Formyl-4-(1,1-dimethylethyl)-6-methylsulfinylphenol

2,8 g (0,01 Mol) 2-Aminomethyl-4-(1,1-dimethylethyl)-6-sulfinylphenolhydrochlorid werden analog Beispiel 1 mit 3 g Urotropin in Trifluoressigsäure umgesetzt. Das Gemisch wird jedoch wie folgt aufgearbeitet : Man gießt das Reaktionsgemisch auf Eiswasser, nimmt die ausgefallene, gummiartige Masse in Diethylether auf und wäscht mit Wasser neutral. Nach Abdampfen des Lösemittels wird der Rückstand der Chromatographie an Kieselgel mit dem Elutionsmittel Toluol/Ethylacetat 4 : 1 unterworfen.
Fahlgelbe Kristalle vom Schmp. : 100-102 °C.

Anstelle von 2-Aminomethyl-4-(1,1-dimethylethyl)-6-methylsulfinylphenolhydrochlorid kann auch das 4-(1,1-dimethylethyl)-2-methylsulfinylphenol eingesetzt werden.

Herstellung der Ausgangsverbindung :

a) 4-(1,1-dimethylethyl)-2-(methylsulfinyl) phenol

52 g (0,27 Mol) 4-(1,1-Dimethylethyl)-2-hydroxythioanisol werden in 300 ml Eisessig gelöst. Unter Eiskühlung werden 30 ml 30 % $H_2O_2$ zugetropft. Man rührt 2 Stunden bei Raumtemperatur, geißt auf Eiswasser und saugt ab. Umkristallisation aus Toluol.
Weiße Kristalle vom Schmp. : 149-150 °C.

b) [5-(1,1-Dimethylethyl)-2-hydroxy-3-methylsulfinylbenzyl] trimethylammoniumjodid

11 g (0,052 Mol) 4-(1,1-dimethylethyl)-2-methylsulfinylphenol werden mit 14 ml (0,1 Mol) 40 %iger wäßriger Dimethylaminlösung und 10 ml 35 %iger wäßriger Formaldehydlösung (0,1 Mol) in 100 ml Ethanol 1 Stunde am Rückfluß gekocht. Man entfernt das Lösungsmittel am Rotationsverdampfer, nimmt in 2 N Salzsäure auf und extrahiert mit Ethylacetat. Die wäßrige Phase wird erneut bis zur Trockne eingedampft, der Rückstand in Aceton aufgenommen und mit 30 ml Jodmethan versetzt. Man beläßt ca. 1 Stunde bei Raumtemperatur und engt ein bis zur beginnenden Kristallisation.
Weiße Kristalle vom Schmp. : 183-185 °C.

c) Methyl-[3-azidomethyl-5-(1,1-dimethylethyl)-2-hydroxyphenyl] sulfoxid

8,9 g (0,022 Mol) des unter b) erhaltenen Ammoniumjodids werden in 80 ml Dimethylformamid gelöst, mit 5 g (0,08 Mol) Natriumazid versetzt und 30 Minuten bei 100 °C gerührt. Anschließend gießt man auf Eiswasser und saugt ab. Das noch schwach feuchte, gelbliche Produkt wird ohne weitere Reinigung in 80 ml Methanol gelöst.

d) 2-Aminomethyl-4-(1,1-dimethylethyl)-6-methylsulfinylphenolhydrochlorid

Die unter c) erhaltene methanolische Lösung von Methyl-[3-azidomethyl-5-(1,1-dimethylethyl)-2-hydroxyphenyl]-sulfoxid wird mit einer Suspension von 1 g 10 % Palladium auf Kohle vereint und 2

Stunden bei Raumtemperatur und Normaldruck hydriert. Man filtriert, engt ein und chromatographiert den Rückstand an Kieselgel, wobei ein Gemisch aus Ethylacetat und Methanol (2 : 1) als Elutionsmittel dient. Es fällt zunächst das freie Amin an (Schmp. : 192-193 °C), das durch Umkristallisation aus 2 N Salzsäure in die Titelverbindung übergeht.

Weiße Kristalle vom Schmp. : 84-86 °C.

Beispiel 10

2-Formyl-4-chlor-6-methylsulfonylphenol

2,4 g (0,009 Mol) 2-Aminomethyl-4-chlor-6-methylsulfonylphenolhydrochlorid werden mit 1,6 g Urotropin in 18 ml Trifluoressigsäure zum 2-Formyl-4-chlor-6-methylsulfonylphenol analog Beispiel 1 umgesetzt.

Herstellung der Ausgangsverbindung :

Das 2-Aminomethyl-4-chlor-6-methylsulfonylphenolhydrochlorid wird analog der Reaktionsfolge 1 a bis 1 f hergestellt, jedoch mit 4-Chloranisol anstelle von 4-(1,1-Dimethylethyl) anisol in Stufe 1 a.

Schmp. : 153-154 °C.

Beispiel 11

2-Acetyl-4-(1,1-dimethylethyl)-6-methylsulfonylphenol

256 mg (1 mMol) des in Beispiel 1 erhaltenen 2-Formyl-4-(1,1-dimethylethyl)-6-methylsulfonylphenols werden in 10 ml absolutem Diethylether gelöst, mit 328 mg (2 mMol) N,N,N',N'-Tetramethylethylendiamin versetzt sowie unter Stickstoff mit 1 ml einer 2,9 molaren Lösung von Methylmagnesiumbromid in Diethylether. Man erhitzt 30 Minuten am Rückfluß, gießt auf Eiswasser, trennt die organische Phase ab und wäscht sie neutral. Nach Verdampfen des Lösemittels unterwirft man den Rückstand der Chromatographie an Kieselgel mit dem Elutionsmittel n-Hexan/Ethylacetat 2 : 1. Man erhält das 2-(1-Hydroxyethyl)-4-(1,1-dimethylethyl)-6-methylsulfonylphenol vom Schmp. 90 °C, welches sofort in 2 ml Diethylether gelöst wird. Man gibt 1 ml einer Lösung von 5 g $Na_2Cr_2O_7 \cdot 2H_2O$ sowie 3,75 ml $H_2SO_4$ in 25 ml $H_2O$ dazu und rührt 2 Stunden bei Zimmertemperatur. Man gießt auf Wasser, trennt die organische Phase ab, wäscht sie neutral und dampft das Lösemittel im Vakuum ab. Der Rückstand wird an Kieselgel mit dem Elutionsmittel Ethylacetat/Toluol 4 : 1 chromatographiert.

Weiße Kristalle vom Schmp. : 138-140 °C.

Beispiel 12

2-(2,5-Dimethyl-1-imidazolidinyl)-4-(1,1-dimethylethyl)-6-methylsulfonylphenol

1,28 g (0,005 Mol) des Unter Beispiel 1 erhaltenen 2-Formyl-4-(1,1-dimethylethyl)-6-methylsulfonylphenols werden in 20 ml Methylenchlorid gelöst und mit 0,44 g (0,523 Mol) N,N'-Dimethylethylendiamin versetzt. Man rührt 1 h bei Zimmertemperatur und dampft dann das Lösemittel ab. Der Rückstand wird mit Ether kristallisiert.

Weiße Kristalle vom Schmp. : 139-140 °C.

Beispiel 13

2-Formyl-4-(1,1-dimethylethyl)-6-sulfamoylphenol

2,84 g (0,01 Mol) 4-(1,1-dimethylethyl)-2-dimethylaminomethylenaminosulfonylphenol werden mit 1,4 g Urotropin in 10 ml Trifluoressigsäure 6 h am Rückfluß gekocht. Man gießt auf Eiswasser, nimmt die ausgefallene, gummiartige Masse in Ether auf, wäscht neutral und verdampft das Lösemittel im Vakuum. Der Rückstand wird an Kieselgel mit Ethylacetat/Toluol 4 : 1 als Elutionsmittel chromatographiert. Man erhält das 2-Formyl-4-(1,1-dimethylethyl)-6-dimethylaminomethylenaminosulfonylphenol vom Schmp. 182-183 °C, das sofort in 2 N Natronlauge gelöst wird. Man beläßt 30 Minuten auf dem Dampfbad, säuert die Lösung mit 2 N Salzsäure auf pH 2-3 an und saugt ab.

Fahlgelbe Kristalle vom Schmp. : 147-148 °C.

Herstellung der Ausgangsverbindung 4-(1,1-dimethylethyl)-2-dimethylaminomethylenaminosulfonylphenol

a) 3-(1,1-Dimethylethyl)-6-methoxybenzolsulfonsäurechlorid

12,3 g (0,075 Mol) 4-(1,1-Dimethylethyl) anisol gelöst in 30 ml Methylenchlorid tropft man unter Eiskühlung zu 16,5 ml Chlorsulfonsäure gelöst in 20 ml Methylenchlorid. Man rührt das Gemisch 40 Minuten und gießt anschließend auf Eiswasser. Die organische Phase wird abgetrennt, mit Wasser gewaschen, mit $MgSO_4$ getrocknet und eingeengt. Umkristallisation aus Toluol/Petrolether liefert Kristalle vom Schmp. : 75-77 °C.

b) 3-(1,1-Dimethylethyl)-6-methoxybenzolsulfonsäureamid

25,3 g (0,1 Mol) 3-(1,1-Dimethylethyl)-6-methoxybenzolsulfonsäurechlorid werden in wenig Aceton gelöst und bei Raumtemperatur langsam in 100 ml konz. Ammoniaklösung eingetropft. Man rührt 30 Minuten bei Raumtemperatur und gießt auf Eiswasser. Nach Ansäuern mit konz. Salzsäure wird abgesaugt. Die Umkristallisation erfolgt aus Isopropanol.
Weiße Kristalle vom Schmp. : 156-158 °C.

c) 4-(1,1-Dimethylethyl)-2-dimethylaminomethylenaminosulfonylanisol

4,68 g (0,02 Mol) 3-(1,1-Dimethylethyl)-6-methoxybenzolsulfonsäureamid werden in 50 ml Dimethylformamid gelöst und mit 2,5 g (0,022 Mol) Dimethylformamiddimethylacetal versetzt. Man beläßt 30 Minuten bei Raumtemperatur, gießt auf Eiswasser und saugt ab.
Weiße Kristalle vom Schmp. : 134-136 °C.

d) 4-(1,1-Dimethylethyl)-2-dimethylaminomethylenaminosulfonylphenol

2,98 g (0,01 Mol) 4-(1,1-Dimethylethyl-2-dimethylaminomethylenaminosulfonylanisol werden in 30 ml Methylenchlorid gelöst und mit 2,75 g (0,011 Mol) Bortribromid versetzt. Man rührt 45 Minuten bei Raumtemperatur und zerstört überschüssiges Bortribromid vorsichtig durch Zugabe von Methanol. Man entfernt das Lösemittelgemisch im Vakuum und verreibt den Rückstand mit Wasser. Das Produkt fällt in Form leicht gelblicher Kristalle an. Die Umkristallisation geschieht aus Isopropanol.
Schmp. 162-164 °C.

Beispiel 14

2-Formyl-4-methyl-6-methylsulfonylphenol

1,89 g 2-Aminomethyl-4-methyl-6-methylsulfonylphenolhydrochlorid werden mit 1,35 g Urotropin in 15 ml Trifluoressigsäure analog Beispiel 1 umgesetzt.
Fahlgelbe Kristalle vom Schmp. : 150-151 °C.
Das Ausgangsmaterial 2-Aminomethyl-4-methyl-6-methylsulfonylphenolhydrochlorid wird analog der Reaktionsfolge 1 a-1 f hergestellt.
Schmp. : 227-228 °C.

Beispiel 15

2-Formyl-4-(1-methylpropyl)-6-methylsulfonylphenol

0,48 g (0,001 6 Mol) 2-Aminomethyl-4-(1-methylpropyl)-6-methylsulfonylphenolhydrochlorid werden mit 0,22 g Hexamethylentetramin in 3 ml Trifluoressigsäure analog Beispiel 1 umgesetzt. Schmp. : 68-70 °C.
Das Ausgangsmaterial 2-Aminomethyl-4-(1-methylpropyl)-6-methylsulfonylphenol wird analog der Reaktionsfolge 1 a-1 f hergestellt. Schmp. : 236-238 °C.

Beispiel 16

2-Formyl-4-(1,1-dimethylethyl)-6-isopropylsulfonylphenol

0,4 g (0,001 2 Mol) 2-Aminomethyl-4-(1,1-dimethylethyl)-6-isopropylsulfonylphenolhydrochlorid werden analog Beispiel 1 mit 0,17 g Hexamethylentetramin in 2,5 ml Trifluoressigsäure umgesetzt. Schmp. : 128-130 °C.
Das Ausgangsmaterial 2-Aminomethyl-4-(1,1-dimethylethyl)-6-isopropylsulfonylphenolhydrochlorid wird analog der Reaktionsfolge 1 a-1 f hergestellt. Schmp. : 209-212 °C.

Beispiel 17

2-Formyl-4-ethyl-6-methylsulfonylphenol

0,79 g (0,003 Mol) 2-Aminomethyl-4-ethyl-6-methylsulfonylphenolhydrochlorid werden mit 0,42 g Hexamethylentetramin in 6 ml Trifluoressigsäure analog Beispiel 1 umgesetzt. Schmp. : 114-116 °C.

Das Ausgangsmaterial 2-Aminomethyl-4-ethyl-6-methylsulfonylphenolhydrochlorid wird analog der Reaktionsfolge 1 a-1 f hergestellt. Schmp. : 101-102 °C.

Beispiel 18

N-Methyl-2-hydroxy-5-(1,1-dimethylethyl)-3-methylsulfonylbenzaldimin

2,56 g (0,01 Mol) 2-Formyl-4-(1,1-dimethylethyl)-6-methylsulfonylphenol werden in 30 ml Methanol gelöst. Unter Rühren wird solange gasförmiges Methylamin eingeleitet bis dünnschichtchromatographisch kein Ausgangsmaterial mehr nachzuweisen ist. Man zieht das Methanol im Vakuum ab und kristallisiert den öligen Rückstand aus Diethylether/Petrolether um. Gelbe Kristalle vom Schmp. : 149-151 °C.

Beispiel 19

N-Ethyl-2-hydroxy-5-(1,1-dimethylethyl)-3-methylsulfonylbenzaldimin

2,56 g (0,01 Mol) 2-Formyl-4-(1,1-dimethylethyl)-6-methylsulfonylphenol werden analog Beispiel 18 mit Ethylamin umgesetzt. Gelbe Kristalle vom Schmp. : 87-88 °C.

**Patentansprüche** (für die Vertragsstaaten : BE, CH, DE, FR, GB, IT, LI, LU, NL, SE)

1. Verbindung der allgemeinen Formel I

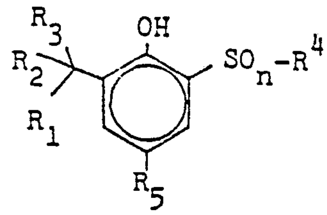

(I)

in welcher

$R^1$ für Wasserstoff, Alkyl mit 1 bis 4 C-Atomen, $OR^7$, wobei $R^7$ Wasserstoff oder Alkyl mit 1-4 C-Atomen bedeutet, oder $NR^8R^9$, wobei $R^8$ und $R^9$ gleich oder verschieden sind und Wasserstoff oder Alkyl mit 1 bis 4 C-Atomen bedeuten, steht ;

$R^2$ und $R^3$ gleich oder verschieden sind und für $OR^{10}NR^{10}R^{11}SR^{10}$ stehen, wobei $R^{10}$ und $R^{11}$ gleich oder verschieden sind und für Alkyl mit 1 bis 8 C-Atomen stehen oder

$R^2$ und $R^3$ gemeinsam eine Kette $—X—(CH_2)_m—X—$ bilden, wobei X für O, S oder $NR^{11}$ steht und m 2 bis 6 bedeutet, oder gemeinsam für eine Carbonylfunktion = O oder eine Iminfunktion $=N—R^{11}$ stehen ;

$R^4$ für Alkyl mit 1 bis 4 C-Atomen, Cycloalkyl mit 3 bis 6 C-Atomen, Alkenyl mit 2 bis 4 C-Atomen und bis zu 2 Doppelbindungen und jeweils bis zu 3 Halogenatomen oder für $NR^{12}R^{13}$ steht, wobei $R^{12}$ und $R^{13}$ gleich oder verschieden sind und Wasserstoff oder Alkyl mit 1 bis 4 C-Atomen bedeuten ;

$R^5$ für Alkyl mit 1 bis 10 C-Atomen oder Cycloalkyl mit 3 bis 8 C-Atomen und bis zu 8 Ringgliedern oder für Halogen steht ; und

n 1 oder 2 beträgt,

jedoch mit Ausnahme von 2-Hydroxy-5-methyl-3-methylsulfinylacetophenon, 2-Hydroxy-5-methyl-3-methylsulfonylacetophenon, 5-Chlor-3-methylsulfonylsalicylsäure, 3-Brom-5-methylsulfonylsalicylsäure und von 5-Brom-3-n-butyl-sulfonylsalicylsäure.

2. Verbindung nach Anspruch 1, dadurch gekennzeichnet, daß

$R^1$ für Wasserstoff oder OH steht ;

$R^2$, $R^3$ gemeinsam eine Carbonylfunktion oder eine Kette

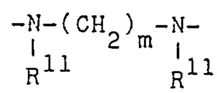

bilden, wobei m=2 oder 3 ist und $R^{11}$ Methyl oder Ethyl bedeuten ;

$R^4$ Methyl oder $NH_2$ bedeutet ;

$R^5$ Alkyl mit 4 bis 8 C-Atomen bedeutet
n 1 oder 2 bedeutet.

3. Verbindung nach Anspruch 2, dadurch gekennzeichnet, daß $R^1$ für Wasserstoff, $R^2$ und $R^3$ gemeinsam für die Carbonylfunktion, $R^4$ für Methyl, $R^5$ für Alkyl mit 4 bis 8 C-Atomen und n für 1 oder 2 stehen.

4. Verbindung nach Anspruch 3, dadurch gekennzeichnet, daß $R^5$ 1,1-Dimethylethyl und n 2 bedeuten.

5. Verbindung nach Anspruch 3, dadurch gekennzeichnet, daß $R^5$ 1,1-Dimethylethyl und n 1 sind.

6. Verbindung nach Anspruch 1, dadurch gekennzeichnet, daß $R^1$ für Wasserstoff, $R^2$ und $R^3$ gemeinsam für die Carbonylfunktion, $R^4$ für Chlormethyl, $R^5$ für 1,1-Dimethylethyl und n für 2 stehen.

7. Verbindung nach Anspruch 2, dadurch gekennzeichnet, daß $R^1$ für Wasserstoff, $R^2$ und $R^3$ gemeinsam für die Carbonylfunktion, $R^4$ für $NH_2$, $R^5$ für 1,1-Dimethylethyl und n für 2 stehen.

8. Verbindung nach Anspruch 2, dadurch gekennzeichnet, daß $R^1$ für H, $R^2$ und $R^3$ gemeinsam für

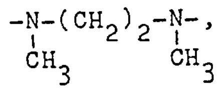

$R^4$ für Methyl, $R^5$ für 1,1-Dimethylethyl und n für 2 stehen.

9. Verbindung nach Anspruch 1, dadurch gekennzeichnet, daß
$R^1$ für Wasserstoff oder OH steht ;
$R^2$ und $R^3$ gemeinsam für die Carbonylfunktion = O stehen ;
$R^4$ $NR^{12}R^{13}$ bedeutet, wobei $R^{12}$ und $R^{13}$ gleich oder verrschieden sind und Wasserstoff oder Alkyl mit 1-4 C-Atomen bedeuten ; und daß
$R^5$ für Alkyl mit 3 bis 8 C-Atomen ; und
n für 2 steht.

10. Verfahren zum Herstellen einer Verbindung der allgemeinen Formel I nach Anspruch 1, dadurch gekennzeichnet, daß man

a) Verbindungen der Formel II

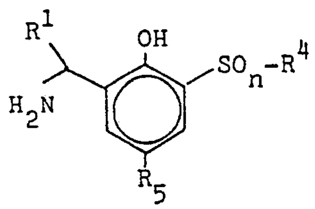

(II)

in der $R^1$ Wasserstoff oder $C_1$-$C_4$-Alkyl, $R^4$, $R^5$ und n die obengenannten Bedeutungen aufweisen, $R^4$ jedoch zusätzlich auf für eine Gruppierung —N=Z stehen kann, wobei Z die Bedeutung einer Schutzgruppe der Formel V zukommt,

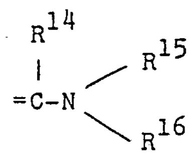

(V)

in der die Reste $R^{14}$ Wasserstoff oder Alkyl mit 1 bis 4 C-Atomen und $R^{15}$, $R^{16}$ Alkyl mit 1 bis 4 C-Atomen bedeuten, mit Formaldehyd oder einem Formaldehyd erzeugenden Reagenz umsetzt zu Verbindungen der Formel I und gegebenenfalls die Schutzgruppe Z hydrolytisch abspaltet ;

b) Verbindungen der allgemeinen Formel III

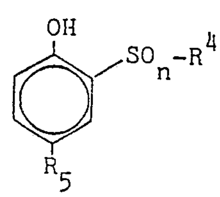

(III)

in der $R^4$, $R^5$ und n die obengenannten Bedeutungen haben, $R^4$ jedoch zusätzlich die unter Verfahrensvariante a) definierte Bedeutung —N=Z aufweisen kann, umsetzt mit einem Formylierungsreagens zu Verbindungen der allgemeinen Formel I und gegebenenfalls die Schutzgruppe Z hydrolytisch

abspaltet ;

c) Verbindungen der allgemeinen Formel IV

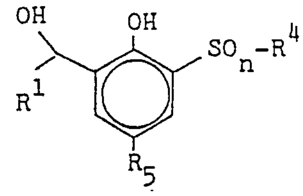

(IV)

RESERVE 2 QG

in der $R^1$, $R^4$, $R^5$ und n die genannten Bedeutungen haben, $R^4$ jedoch zusätzlich die unter Verfahrensvariante a) definierte Bedeutung —N=Z aufweisen kann, oxidiert zu Verbindungen der allgemeinen Formel I und gegebenenfalls die Schutzgruppe Z hydrolytisch abspaltet ;

d) Verbindungen der Formel I, in der $R^1$ für Wasserstoff, $R^2$, $R^3$ gemeinsam für die Carbonylfunktion stehen, $R^4$ und $R^5$ die obengenannten Bedeutungen haben, $R^4$ jedoch zusätzlich die unter Verfahrensvariante a) beschriebene Bedeutung —N=Z haben kann, oxidiert zu Verbindungen der allgemeinen Formel I und gegebenenfalls die Schutzgruppe Z hydrolytisch abspaltet und gegebenenfalls die erhaltenen Säuren in ihre Ester oder Amide der allgemeinen Formel I überführt ;

e) Verbindungen der allgemeinen Formel I, in der $R^1$ für Wasserstoff oder Alkyl mit 1-4 C-Atomen steht ; $R^2$, $R^3$ gemeinsam eine Carbonylfunktion bilden ; $R^4$, $R^5$ und n die obengenannten Bedeutungen haben, mit Alkoholen $R^{10}OH$, Thioalkoholen $R^{10}SH$, Aminen $NHR^{10}R^{11}$ sowie mit Verbindungen $HX—(CH_2)_m—XH$, wobei $R^{10}$, $R^{11}$, X und m die obengenannten Bedeutungen haben, in die entsprechenden Acetale, Thioacetale, Aminale oder Imine der allgemeinen Formel I überführt.

f) Verbindungen der allgemeinen Formel XVII

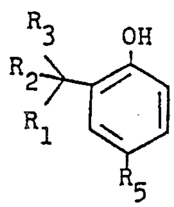

(XVII)

chlorsulfoniert und die entstandenen Sulfochloride

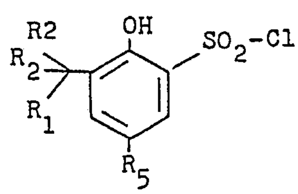

(XVIII)

entweder mit Aminen

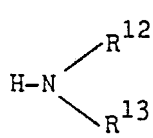

umsetzt zu den Verbindungen I oder einer Reduktion zu den Sulfinsäuren XIX

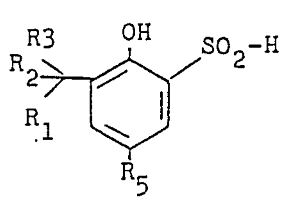

(XIX)

unterwirft und diese durch Alkylierung in die Verbindungen I überführt.

11. Verbindung der allgemeinen Formel I gemäß Anspruch 1 zur Behandlung und Prophylaxe von Schädigungen der Schleimhaut des Magen-Darmtraktes, und von Schädigungen der Leber, des Pankreas

und des Gefäßsystems.

**Patentansprüche** (für den Vertragsstaat AT)

1. Verfahren zum Herstellen einer Verbindung der allgemeinen Formel I

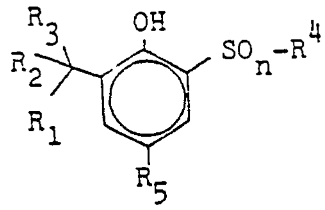

(I)

in welcher

$R^1$ für Wasserstoff, Alkyl mit 1 bis 4 C-Atomen, $OR^7$, wobei $R^7$ Wasserstoff oder Alkyl mit 1 bis 4 C-Atomen bedeutet, oder $NR^8R^9$, wobei $R^8$, $R^9$ gleich oder verschieden sind und Wasserstoff oder Alkyl mit 1 bis 4 C-Atomen bedeuten, steht ;

$R^2$, $R^3$ gleich oder verschieden sind und für $OR^{10}$, $NR^{10}R^{11}$ oder $SR^{10}$ stehen, wobei $R^{10}$ und $R^{11}$ gleich oder verschieden sind und für Alkyl mit 1 bis 8 C-Atomen stehen oder

$R^2$, $R^3$ gemeinsam eine Kette $-X-(CH_2)_m-X-$ bilden, wobei X für O, S oder $NR^{11}$ steht und m 2 bis 6 bedeutet, oder

$R^2$, $R^3$ gemeinsam für eine Carbonylfunktion =O oder eine Iminfunktion $=N-R^{11}$ stehen ;

$R^4$ für Alkyl mit 1 bis 4 C-Atomen, Cycloalkyl mit 3 bis 6 C-Atomen, Alkenyl mit 2 bis 4 C-Atomen und bis zu 2 Doppelbindungen und jeweils bis zu 3 Halogenatomen oder für $NR^{12}R^{13}$ steht, wobei $R^{12}$ und $R^{13}$ gleich oder verschieden sind und Wasserstoff oder Alkyl mit 1 bis 4 C-Atomen bedeuten ;

$R^5$ für Alkyl mit 1 bis 10 C-Atomen oder Cycloalkyl mit 3 bis 8 C-Atomen und bis zu 8 Ringgliedern oder für Halogen steht ; und

n 1 oder 2 beträgt,

jedoch mit Ausnahme von 2-Hydroxy-5-methyl-3-methylsulfinylacetophenon, 2-Hydroxy-5-methyl-3-methylsulfonylacetophenon, 5-Chlor-3-methylsulfonylsalicylsäure, 3-Brom-5-methylsulfonylsalicylsäure und von 5-Brom-3-n-butyl-sulfonylsalicylsäure,

dadurch gekennzeichnet, daß man

a) Verbindungen der Formel II

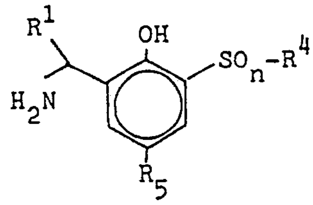

(II)

in der $R^1$ Wasserstoff oder $C_1$-$C_4$-Alkyl, $R^4$, $R^5$ und n die obengenannten Bedeutungen aufweisen, $R^4$ jedoch zusätzlich auch für eine Gruppierung —N=Z stehen kann, wobei Z die Bedeutung einer Schutzgruppe der Formel V zukommt,

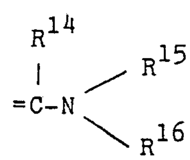

(V)

in der die Reste $R^{14}$ Wasserstoff oder Alkyl mit 1 bis 4 C-Atomen und $R^{15}$, $R^{16}$ Alkyl mit 1 bis 4 C-Atomen bedeuten, mit Formaldehyd oder einem Formaldehyd erzeugenden Reagenz umsetzt zu Verbindungen der Formel I und gegebenenfalls die Schutzgruppe Z hydrolytisch abspaltet ;

b) Verbindungen der allgemeinen Formel III

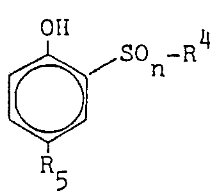

(III)

in der $R^4$, $R^5$ und n die obengenannten Bedeutungen haben, $R^4$ jedoch zusätzlich die unter Verfahrensvariante a) definierte Bedeutung —N=Z aufweisen kann, umsetzt mit einem Formylierungsreagenz zu Verbindungen der allgemeinen Formel I und gegebenenfalls die Schutzgruppe Z hydrolytisch abspaltet ;

c) Verbindungen der allgemeinen Formel IV

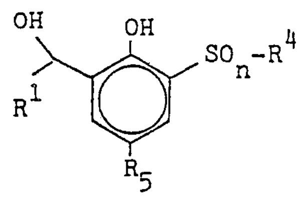

(VI)

in der $R^1$, $R^4$, $R^5$ und n die genannten Bedeutungen haben, $R^4$ jedoch zusätzlich die unter Verfahrensvariante a) definierte Bedeutung —N=Z aufweisen kann, oxidiert zu Verbindungen der allgemeinen Formel I und gegebenenfalls die Schutzgruppe Z hydrolytisch abspaltet ;

d) Verbindungen der Formel I, in der $R^1$ für Wasserstoff, $R^2$, $R^3$ gemeinsam für die Carbonylfunktion stehen, $R^4$ und $R^5$ die obengenannten Bedeutungen haben, $R^4$ jedoch zusätzlich die unter Verfahrensvariante a) beschriebene Bedeutung —N=Z haben kann, oxidiert zu Verbindungen der allgemeinen Formel I und gegebenenfalls die Schutzgruppe Z hydrolytisch abspaltet und gegebenenfalls die erhaltenen Säuren in ihre Ester oder Amide der allgemeinen Formel I überführt.

e) Verbindungen der allgemeinen Formel I, in der $R^1$ für Wasserstoff oder Alkyl mit 1-4 C-Atomen steht ; $R^2$, $R^3$ gemeinsam eine Carbonylfunktion bilden ; $R^4$, $R^5$ und n die obengenannten Bedeutungen haben, mit Alkoholen $R^{10}OH$, Thioalkoholen $R^{10}SH$, Aminen $NHR^{10}R^{11}$ sowie mit Verbindungen $HX—(CH_2)_m—XH$, wobei $R^{10}$, $R^{11}$, X und m die obengenannten Bedeutungen haben, in die entsprechenden Acetale, Thioacetale, Aminale oder Imine der allgemeinen Formel I überführt.

f) Verbindungen der allgemeinen Formel XVII

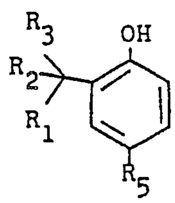

(XVII)

chlorsulfoniert und die entstandenen Sulfochloride

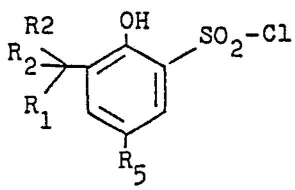

(XVIII)

entweder mit Aminen

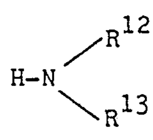

umsetzt zu den Verbindungen einer Reduktion zu den Sulfinsäuren XIX

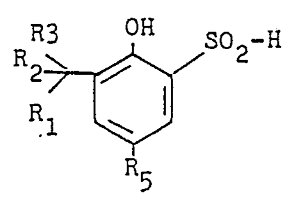

(XIX)

unterwirft und diese durch Alkylierung in die Verbindungen I überführt.

2. Verfahren nach Anspruch 1, dadurch gekennzeichnet, daß
$R^1$ für Wasserstoff oder OH steht ;
$R^2$, $R^3$ gemeinsam eine Carbonylfunktion oder eine Kette

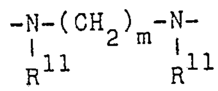

bilden, wobei m = 2 oder 3 ist und $R^{11}$ Methyl oder Ethyl bedeuten ;

$R^4$ Methyl oder $NH_2$ bedeutet ;

$R^5$ Alkyl mit 4 bis 8 C-Atomen bedeutet

n 1 oder 2 bedeutet.

3. Verfahren nach Anspruch 2, dadurch gekennzeichnet, daß $R^1$ für Wasserstoff, $R^2$ und $R^3$ gemeinsam für die Carbonylfunktion, $R^4$ für Methyl, $R^5$ für Alkyl mit 4 bis 8 C-Atomen und n für 1 oder 2 stehen.

4. Verfahren nach Anspruch 3, dadurch gekennzeichnet, daß $R^5$ 1,1-Dimethylethyl und n 2 bedeuten.

5. Verfahren nach Anspruch 3, dadurch gekennzeichnet, daß $R^5$ 1,1-Dimethylethyl und n 1 sind.

6. Verfahren nach Anspruch 1, dadurch gekennzeichnet, daß $R^1$ für Wasserstoff, $R^2$ und $R^3$ gemeinsam für die Carbonylfunktion, $R^4$ für Chlormethyl, $R^5$ für 1,1-Dimethylethyl und n für 2 stehen.

7. Verfahren nach Anspruch 2, dadurch gekennzeichnet, daß $R^1$ für Wasserstoff, $R^2$ und $R^3$ gemeinsamt für die Carbonylfunktion, $R^4$ für $NH_2$, $R^5$ für 1,1-Dimethylethyl und n für 2 stehen.

8. Verfahren nach Anspruch 2, dadurch gekennzeichnet, daß $R^1$ für H, $R^2$ und $R^3$ gemeinsam für

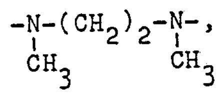

$R^4$ für Methyl, $R^5$ für 1,1-Dimethylethyl und n für 2 stehen.

9. Verfahren nach Anspruch 1, dadurch gekennzeichnet, daß $R^1$ für Wasserstoff oder OH steht ; $R^2$ und $R^3$ gemeinsam für die Carbonylfunktion =O stehen ; $R^4$ $NR^{12}R^{13}$ bedeutet, wobei $R^{12}$ und $R^{13}$ gleich oder verschieden sind und Wasserstoff oder Alkyl mit 1-4 C-Atomen bedeuten ; und daß $R^5$ für Alkyl mit 3 bis 8 C-Atomen ; und n für 2 steht.

**Claims** (for the Contracting States : BE, CH, DE, FR, GB, IT, LI, LU, NL, SE)

1. A compound of the formula I

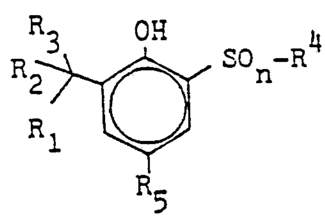

(I)

in which

$R^1$ represents hydrogen, alkyl having 1 to 4 carbon atoms, $OR^7$, where $R^7$ denotes hydrogen or alkyl having 1-4 carbon atoms, or $NR^8R^9$, where $R^8$ and $R^9$ are identical or different and denote hydrogen or alkyl having 1 to 4 carbon atoms ;

$R^2$ and $R^3$ are identical or different and represent $OR^{10}$, $NR^{10}R^{11}$ or $SR^{10}$, where $R^{10}$ and $R^{11}$ are identical or different and represent alkyl having 1 to 8 carbon atoms, or

$R^2$ and $R^3$ together form a chain $—X—(CH_2)_m—X—$, where X represents O, S or $NR^{11}$, and m denotes 2 to 6, or together represent a carbonyl function =O or an imine function $=N—R^{11}$ ;

$R^4$ represents alkyl having 1 to 4 carbon atoms, cycloalkyl having 3 to 6 carbon atoms, alkenyl having 2 to 4 carbon atoms and up to 2 double bonds, and each having up to 3 halogen atoms, or represents $NR^{12}R^{13}$, where $R^{12}$ and $R^{13}$ are identical or different and denote hydrogen or alkyl having 1 to 4 carbon atoms ;

$R^5$ represents alkyl having 1 to 10 carbon atoms or cycloalkyl having 3 to 8 carbon atoms and up to 8 ring members, or represents halogen ; and

n is 1 or 2, but with the exception of 2-hydroxy-5-methyl-3-methylsulfinylacetophenone, 2-hydroxy-5-methyl-3-methylsulfonylacetophenone, 5-chloro-3-methylsulfonylsalicylic acid, 3-bromo-5-methylsulfonylsalicylic acid and of 5-bromo-3-n-butyl-sulfonylsalicyclic acid.

2. A compound as claimed in claim 1, wherein

$R^1$ represents hydrogen or OH ;

$R^2$ and $R^3$ together form a carbonyl function or a chain

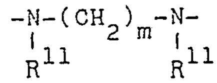

where m = 2 or 3 and $R^{11}$ denotes methyl or ethyl ;

$R^4$ denotes methyl or $NH_2$ ;

$R^5$ denotes alkyl having 4 to 8 carbon atoms ; and

n denotes 1 or 2.

3. A compound as claimed in claim 2, wherein $R^1$ represents hydrogen, $R^2$ and $R^3$ together represent the carbonyl function, $R^4$ represents methyl, $R^5$ represents alkyl having 4 to 8 carbon atoms and n represents 1 or 2.

4. A compound as claimed in claim 3, wherein $R^5$ denotes 1,1-dimethylethyl and n denotes 2.

5. A compound as claimed in claim 3, wherein $R^5$ is 1,1-dimethylethyl and n is 1.

6. A compound as claimed in claim 1, wherein $R^1$ represents hydrogen, $R^2$ and $R^3$ together represent the carbonyl function, $R^4$ represents chloromethyl, $R^5$ represents 1,1-dimethylethyl and n represents 2.

7. A compound as claimed in claim 2, wherein $R^1$ represents hydrogen, $R^2$ and $R^3$ together represent the carbonyl function, $R^4$ represents $NH_2$, $R^5$ represents 1,1-dimethylethyl and n represents 2.

8. A compound as claimed in claim 2, wherein $R^1$ represents H, $R^2$ and $R^3$ together represent

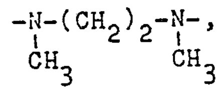

$R^4$ represents methyl, $R^5$ represents, 1,1-dimethylethyl and n represents 2.

9. A compound as claimed in claim 1, wherein

$R^1$ represents hydrogen or OH ;

$R^2$ and $R^3$ together represent the carbonyl function =O ;

$R^4$ denotes $NR^{12}R^{13}$, where $R^{12}$ and $R^{13}$ are identical or different and denote hydrogen or alkyl having 1-4 carbon atoms ; and wherein

$R^5$ represents alkyl having 3 to 8 carbon atoms ; and

n represents 2.

10. A process for the preparation of a compound of the formula I as claimed in claim 1, which comprises

a) reacting a compound of the formula II

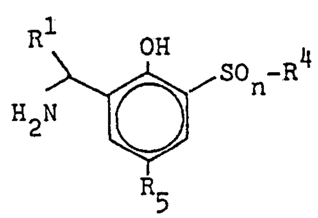

(II)

in which $R^1$ denotes hydrogen or $C_1$-$C_4$-alkyl, $R^4$, $R^5$ and n have the abovementioned meanings, but $R^4$ can also represent a group —N=Z, Z having the meaning of a protective group of the formula V

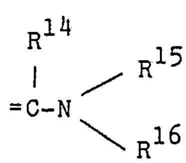

(V)

in which the radicals $R^{14}$ denote hydrogen or alkyl having 1 to 4 carbon atoms, and $R^{15}$ and $R^{16}$ denote alkyl having 1 to 4 carbon atoms, with formaldehyde, or a reagent producing formaldehyde, to give a compound of the formula I and, where appropriate, eliminating the protective group Z by hydrolysis ;

b) reacting a compound of the formula III

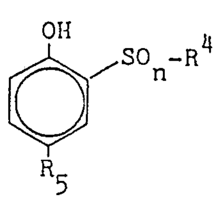

(III)

in which $R^4$, $R^5$ and n have the abovementioned meanings, but $R^4$ can also have the meaning —N = Z defined under process variant a), with a formylating reagent to give a compound of the formula I and, where appropriate, eliminating the protective group Z by hydrolysis ;

c) oxidizing a compound of the formula IV

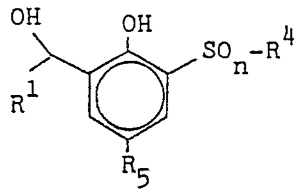

(VI)

in which $R^1$, $R^4$, $R^5$ and n have the abovementioned meanings, but $R^4$ can also have the meaning —N = Z defined under process variant a), to give a compound of the formula I and, where appropriate, eliminating the protective group Z by hydrolysis ;

d) oxidizing a compound of the formula I, in which $R^1$ represents hydrogen, $R^2$ and $R^3$ together represent the carbonyl fucntion, and $R^4$ and $R^3$ together represent the carbonyl function, and $R^4$ and $R^5$ have the abovementioned meanings, but $R^4$ can also have the meaning —N = Z described under process variant a), to give a compound of the formula I and, where appropriate, eliminating the protective group Z by hydrolysis and, where appropriate, converting the resulting acid into its ester or amide of the formula I ;

e) converting a compound of the formula I, in which $R^1$ represents hydrogen or alkyl having 1 to 4 carbon atoms ; $R^2$ and $R^3$ together form a carbonyl function ; and $R^4$, $R^5$ and n have the abovementioned meanings, using an alcohol $R^{10}OH$, a thioalcohol $R^{10}SH$, an amine $NHR^{10}R^{11}$ and using a compound $HX—(CH_2)_m—XH$, $R^{10}$, $R^{11}$, X and m having the abovementioned meanings, into the corresponding acetal, thioacetal, aminal or imine of the formula I ;

f) chlorosulfonating a compound of the formula XVII

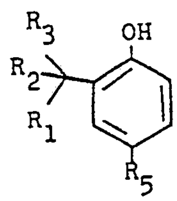

(XVII)

and either reacting the resultant sulfonyl chloride

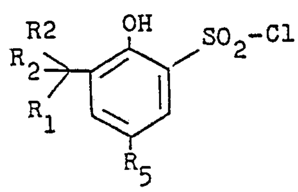

(XVIII)

with an amine

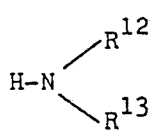

to give a compound I, or subjecting it to reduction to give the sulfinic acid XIX

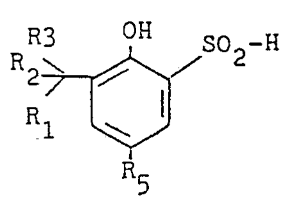

(XIX)

and converting the latter by alkylation into the compound I.

11. A compound of the formula I as claimed in claim 1 for the treatment and prophylaxis of damage to the mucosa of the gastrointestinal tract and of damage to the liver, the pancreas and the vascular system.

**Claims** (for the Contracting State AT)

1. A process for the preparation of a compound of the formula I

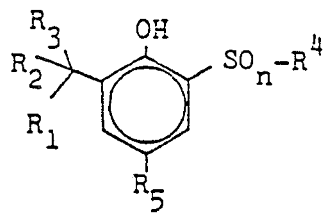

(I)

in which

$R^1$ represents hydrogen, alkyl having 1 to 4 carbon atoms, $OR^7$, where $R^7$ denotes hydrogen or alkyl having 1-4 carbon atoms, or $NR^8R^9$, where $R^8$ and $R^9$ are identical or different and denote hydrogen or alkyl having 1 to 4 carbon atoms ;

$R^2$ and $R^3$ are identical or different and represent $OR^{10}$, $NR^{10}R^{11}$ or $SR^{10}$, where $R^{10}$ and $R^{11}$ are identical or different and represent alkyl having 1 to 8 carbon atoms, or

$R^2$ and $R^3$ together form a chain $—X—(CH_2)_m—X—$, where X represents O, S or $NR^{11}$, and m denotes 2 to 6, or together represent a carbonyl function = O or an imine function $= N—R^{11}$ ;

$R^4$ represents alkyl having 1 to 4 carbon atoms, cycloalkyl having 3 to 6 carbon atoms, alkenyl having 2 to 4 carbon atoms and up to 2 double bonds, and each having up to 3 halogen atoms, or represents $NR^{12}R^{13}$, where $R^{12}$ and $R^{13}$ are identical or different and denote hydrogen or alkyl having 1 to 4 carbon atoms ;

$R^5$ represents alkyl having 1 to 10 carbon atoms or cycloalkyl having 3 to 8 carbon atoms and up to 8 ring members, or represents halogen ; and

n is 1 or 2, but with the exception of 2-hydroxy-5-methyl-3-methylsulfinylacetophenone, 2-hydroxy-5-methyl-3-methylsulfonylacetophenone, 5-chloro-3-methylsulfonylsalicyclic acid, 3-bromo-5-methylsulfonylsalicyclic acid and of 5-bromo-3-n-butylsulfonylsalicylic acid,
which comprises

a) reacting a compound of the formula II

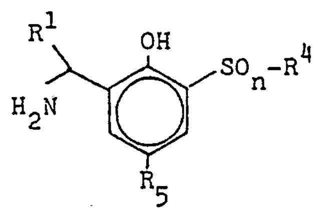

(II)

in which $R^1$ denotes hydrogen or $C_1$-$C_4$-alkyl, $R^4$, $R^5$ and n have the abovementioned meanings, but $R^4$ can also represent a group $—N = Z$, Z having the meaning of a protective group of the formula V

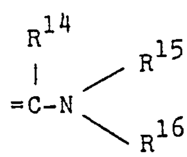

(V)

in which the radicals $R^{14}$ denote hydrogen or alkyl having 1 to 4 carbon atoms, and $R^{15}$ and $R^{16}$ denote alkyl having 1 to 4 carbon atoms, with formaldehyde, or a reagent producing formaldehyde, to give a compound of the formula I and, where appropriate, eliminating the protective group Z by hydrolysis ;

b) reacting a compound of the formula III

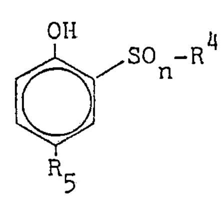

(III)

in which $R^4$, $R^5$ and n have the abovementioned meanings, but $R^4$ can also have the meaning —N = Z defined under process variant a), to give a compound of the formula I and, where appropriate, eliminating the protective group Z by hydrolysis ;

c) oxidizing a compound of the formula IV

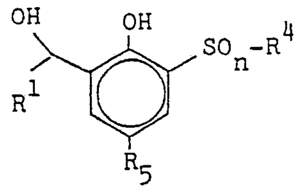

(IV)

in which $R^1$, $R^4$, $R^5$ and n have the abovementioned meanings, but $R^4$ can also have the meaning —N = Z defined under process variant a), to give a compound of the formula I and, where appropriate, eliminating the protective group Z by hydrolysis ;

d) oxidizing a compound of the formula I, in which $R^1$ represents hydrogen, $R^2$ and $R^3$ together represent the carbonyl function, and $R^4$ and $R^5$ have the abovementioned meanings, but $R^4$ and $R^5$ have the abovementioned meanings, but $R^4$ can also have the meaning —N = Z described under process variant a), to give a compound of the formula I and, where appropriate, eliminating the protective group Z by hydrolysis and, where appropriate, converting the resulting acid into its ester or amide of the formula I ;

e) converting a compound of the formula I, in which $R^1$ represents hydrogen or alkyl having 1 to 4 carbon atoms ; $R^2$ and $R^3$ together form a carbonyl function ; and $R^4$, $R^5$ and n have the abovementioned meanings, using an alcohol $R^{10}OH$, a thioalcohol $R^{10}SH$, an amine $NHR^{10}R^{11}$ and using a compound $HX—(CH_2)_m—XH$, $R^{10}$, $R^{11}$, X and m having the abovementioned meanings, into the corresponding acetal, thioacetal, aminal or imine of the formula I ;

f) chlorosulfonating a compound of the formula XVII

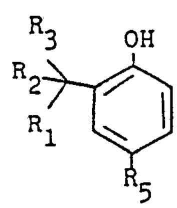

(XVII)

and either reacting the resultant sulfonyl chloride

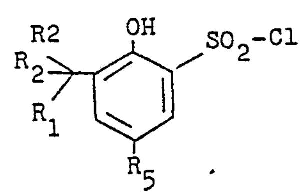

(XVIII)

with an amine

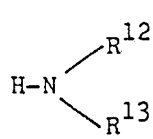

to give a compound (sic) subjecting to a reduction to give the sulfinic acid XIX

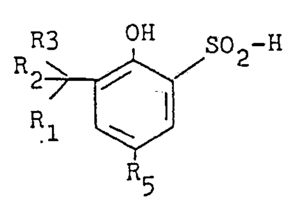

(XIX)

and converting the latter by alkylation into the compound I.

2. A process as claimed in claim 1, wherein

$R^1$ represents hydrogen or OH ;

$R^2$ and $R^3$ together form a carbonyl function or a chain

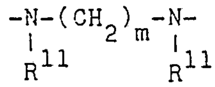

where m = 2 or 3 and $R^{11}$ denotes methyl or ethyl ;

$R^4$ denotes methyl or $NH_2$ ;

$R^5$ denotes alkyl having 4 to 8 carbon atoms ; and n denotes 1 or 2.

3. A process as claimed in claim 2, wherein $R^1$ represents hydrogen, $R^2$ and $R^3$ together represent the carbonyl function, $R^4$ represents methyl, $R^5$ represents alkyl having 4 to 8 carbon atoms and n represents 1 or 2.

4. A process as claimed in claim 3, wherein $R^5$ denotes 1,1-dimethylethyl and n denotes 2.

5. A process as claimed in claim 3, wherein $R^5$ is 1,1-dimethylethyl and n is 1.

6. A process as claimed in claim 1, wherein $R^1$ represents hydrogen, $R^2$ and $R^3$ together represent the carbonyl fucntion, $R^4$ represents chloromethyl, $R^5$ represents 1,1-dimethylethyl and n represents 2.

7. A process as claimed in claim 2, wherein $R^1$ represents hydrogen, $R^2$ and $R^3$ together represent the carbonyl function, $R^4$ represents $NH_2$, $R^5$ represents 1,1-dimethylethyl and n represents 2.

8. A process as claimed in claim 2, wherein $R^1$ represents H, $R^2$ and $R^3$ together represent

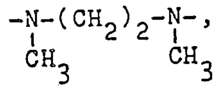

$R^4$ represents methyl, $R^5$ represents 1,1-dimethylethyl and n represents 2.

9. A process as claimed in claim 1, wherein $R^1$ represents hydrogen or OH ; $R^2$ and $R^3$ together represent the carbonyl function = O ; $R^4$ denotes $NR^{12}R^{13}$, where $R^{12}$ and $R^{13}$ are identical or different and denote hydrogen or alkyl having 1-4 carbon atoms ; and wherein $R^5$ represents alkyl having 3 to 8 carbon atoms ; and n represents 2.

**Revendications** (pour les Etats contractants : BE, CH, DE, FR, GB, IT, LI, LU, NL, SE)

1. Composé de formule générale I

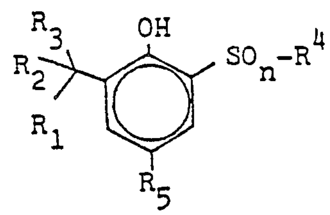

(I)

dans laquelle

$R_1$ représente un atome d'hydrogène, un groupe alkyle à 1 à 4 atomes de C, $OR^7$, $R^7$ représentant un atome d'hydrogène ou un groupe alkyle à 1 à 4 atomes de C, ou $NR^8R^9$, $R^8$ et $R^9$ étant identiques ou différents et représentant un atome d'hydrogène ou un groupe alkyle à 1 à 4 atomes de C ;

$R^2$ et $R^3$ sont identiques ou différents et représentent $OR^{10}$, $NR^{10}R^{11}$ ou $SR^{10}$, $R^{10}$ et $R^{11}$ étant identiques ou différents et représentant un groupe alkyle à 1 à 8 atomes de C ou

$R^2$ et $R^3$ forment ensemble une chaîne $—X—(CH_2)_m—X—$, X représentant O, S ou $NR^{11}$ et m étant un nombre de 2 à 6, ou représentent ensemble une fonction carbonyle = O ou une fonction imine = $N—R^{11}$ ;

$R^4$ représente un groupe alkyle à 1 à 4 atomes de C, cycloalkyle à 3 à 6 atomes de C, alcényle à 2 à 4 atomes de C et comportant jusqu'à 2 doubles liaisons et en tout cas jusqu'à 3 atomes d'halogène ou représente $NR^{12}R^{13}$, $R^{12}$ et $R^{13}$ étant identiques ou différents et représentant un atome d'hydrogène ou un groupe alkyle à 1 à 4 atomes de C ;

$R^5$ représente un groupe alkyle à 1 à 10 atomes de C ou cycloalkyle à 3 à 8 atomes de C et ayant jusqu'à 8 chaînons dans le cycle ou un halogène ; et

n est égal à 1 ou 2,

à l'exception toutefois de la 2-hydroxy-5-méthyl-3-méthylsulfinylacétophénone, de la 2-hydroxy-5-méthyl-3-méthylsulfonylacétophénone, de l'acide 5-chloro-3-méthylsulfonylsalicyclique, de l'acide 3-bromo-5-méthylsulfonylsalicyclique et de l'acide 5-bromo-3-n-butyl-sulfonylsalicyclique.

2. Composé selon la revendication 1 caractérisé en ce que :

$R^1$ représente un atome d'hydrogène ou OH ;

$R^2$, $R^3$ forment ensemble une fonction carbonyle ou une chaîne

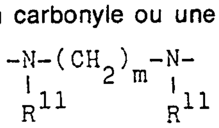

m étant égal à 2 ou 3 et $R^{11}$ signifiant méthyle ou éthyle ;

$R^4$ signifie méthyle ou $NH_2$ ;

$R^5$ signifie un groupe alkyle à 4 à 8 atomes de C, et

n signifie 1 ou 2.

3. Composé selon la revendication 2 caractérisé en ce que $R^1$ représente un atome d'hydrogène, $R^2$ et $R^3$ représentent ensemble la fonction carbonyle, $R^4$ représente un groupe méthyle, $R^5$ un gorupe alkyle à 4 à 8 atomes de C et n représente 1 ou 2.

4. Composé selon la revendication 3 caractérisé en ce que $R^5$ signifie 1,1-diméthyléthyle et n signifie 2.

5. Composé selon la revendication 3 caractérisé en ce que $R^5$ est un groupe 1,1-diméthyléthyle et n est égal à 1.

6. Composé selon la revendication 1 caractérisé en ce que $R^1$ représente un atome d'hydrogène, $R^2$ et $R^3$ représentent ensemble la fonction carbonyle, $R^4$ représente le groupe chlorométhyle, $R^5$ le groupe 1,1-diméthyléthyle et n est égal à 2.

7. Composé selon la revendication 2 caractérisé en ce que $R^1$ représente un atome d'hydrogène, $R^2$ et $R^3$ représentent ensemble la fonction carbonyle, $R^4$ représente $NH_2$, $R^5$ 1,1-diméthyléthyle et n est égal à 2.

8. Composé selon la revendication 2 caractérisé en ce que $R^1$ représente H, $R^2$ et $R^3$ représentent ensemble

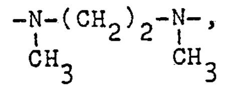

$R^4$ représente méthyle, $R^5$ 1,1-diméthyléthyle et n égale 2.

9. Composé selon la revendication 1 caractérisé en ce que :

$R^1$ représente un atome d'hydrogène ou OH ;

$R^2$ et $R^3$ représentent ensemble la fonction carbonyle =O ;

$R^4$ signifie $NR^{12}R^{13}$, $R^{12}$ et $R^{13}$ étant identiques ou différents et représentant un atome d'hydrogène ou un groupe alkyle à 1 à 4 atomes de C ; et en ce que

$R^5$ représente un groupe alkyle à 3 à 8 atomes de C ; et

n est égal à 2.

10. Procédé de préparation d'un composé de formule générale I selon la revendication 1 caractérisé en ce que

a) on fait réagir des composés de formule II

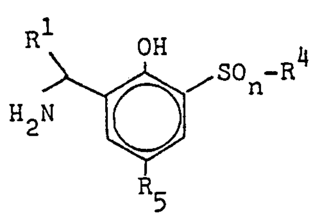

(II)

dans laquelle $R^1$ représente un atome d'hydrogène ou un groupe alkyle à 1 à 4 atomes de C, $R^4$, $R^5$ et n ont les significations indiquées ci-dessus, $R^4$ cependant pouvant aussi représenter un groupement —N = Z, Z ayant la signification d'un groupe protecteur de formule V

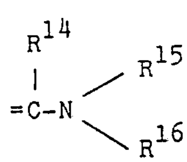

(V)

dans laquelle les radicaux $R^{14}$ désignent un atome d'hydrogène ou un groupe alkyle à 1 à 4 atomes de C et $R^{15}$, $R^{16}$ désignent un groupe alkyle à 1 à 4 atomes de C, avec du formaldéhyde ou un réactif donnant naissance à du formaldéhyde pour obtenir des composés de formule I et éventuellement on sépare le groupe protecteur Z par hydrolyse ;

b) on fait réagir des composés de formule III

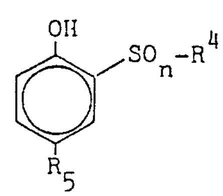

(III)

dans laquelle $R^4$, $R^5$ et n ont les significations indiquées ci-dessus, $R^4$ cependant pouvant avoir en outre la signification $—N = Z$ définie dans la variante a) du procédé, avec un réactif de formylation pour obtenir des composés de formule générale I et éventuellemnt on sépare le groupe protecteur Z par hydrolyse ;

c) on oxyde des composés de formule générale IV

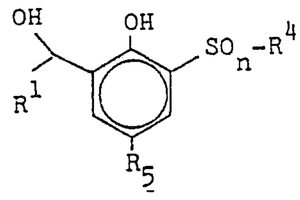

(IV)

dans laquelle $R^1$, $R^4$, $R^5$ et n ont les significations indiquées, $R^4$ cependant pouvant avoir en outre la signification $—N = Z$ définie dans la variante a) du procédé, pour obtenir des composés de formule générale I et éventuellement on sépare le groupe protecteur Z par hydrolyse ;

d) on oxyde des composés de formule I dans laquelle $R^1$ représente un atome d'hydrogène, $R^2$ et $R^3$ représentent ensemble la fonction carbonyle, $R^4$ et $R^5$ ont les significations indiquées ci-dessus, $R^4$ cependant pouvant avoir aussi la signification $—N = Z$ définie dans la variante du procédé a), pour obtenir des composés de formule générale I et éventuellement on sépare le groupe protecteur Z par hydrolyse et éventuellement on transforme les acides obtenus en leurs esters ou amides de formule générale I ;

e) on transforme des composés de formule générale I dans laquelle $R^1$ représente un atome d'hydrogène ou un groupe alkyle à 1 à 4 atomes de C ; $R^2$, $R^3$ forment ensemble une fonction carbonyle ; $R^4$, $R^5$ et n ont les significations indiquées ci-dessus, avec des alcools $R^{10}OH$, des thioalcools $R^{10}SH$, des amines $NHR^{10}R^{11}$ ainsi que des composés $HX—(CH_2)_m—XH$, $R^{10}$, $R^{11}$, X et m ayant les significations indiquées ci-dessus, en les acétals, les thioacétals, aminals ou imines correspondants de formule générale I ;

f) on chlorosulfone des composés de formule générale XVII

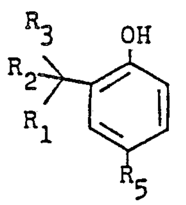

(XVII)

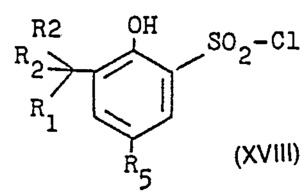

(XVIII)

et on fait réagir les sulfochlorures XVIII formés avec des amines

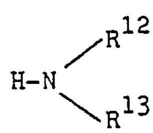

pour donner les composés I ou on les soumet à une réduction pour donner les acides sulfiniques XIX

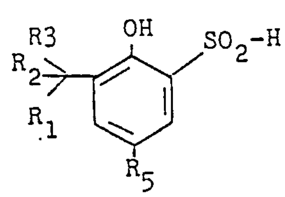

(XIX)

et on transforme ceux-ci par alkylation en les composés I.

11. Composé de formule générale I selon la revendication 1 pour le traitement et la prophylaxie des lésions de la muqueuse du tractus gastro intestinal et de lésions du foie, du pancréas et du système vasculaire.

**Revendications** (pour l'Etat contractant AT)

1. Procédé de préparation d'un composé de formule générale 1

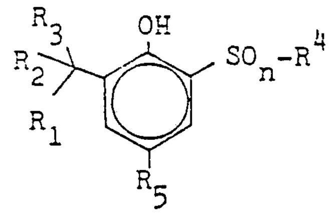

(I)

dans laquelle

$R_1$ représente un atome d'hydrogène, un groupe alkyle à 1 à 4 atomes de C, $OR^7$, $R^7$ représentant un atome d'hydrogène ou un groupe alkyle à 1 à 4 atomes de C, ou $NR^8R^9$, $R^8$, $R^9$ étant identiques ou différents et représentant un atome d'hydrogène ou un groupe alkyle à 1 à 4 atomes de C ;

$R^2$, $R^3$ sont identiques ou différents et représentent $OR^{10}$, $NR^{10}R^{11}$ ou $SR^{10}$, $R^{10}$ et $R^{11}$ étant identiques ou différents et représentant un groupe alkyle à 1 à 8 atomes de C ou

$R^2$, $R^3$ forment ensemble une chaîne $—X—(CH_2)_m—X—$, X représentant O, S ou $NR^{11}$ et m étant un nombre de 2 à 6, ou

$R^2$, $R^3$ représentent ensemble une fonction carbonyle = O ou une fonction imine = $N—R^{11}$ ;

$R^4$ représente un groupe alkyle à 1 à 4 atomes de C, cycloalkyle à 3 à 6 atomes de C, alcényle à 2 à 4 atomes de C et comportant jusqu'à 2 doubles liaisons et en tout cas jusqu'à 3 atomes d'halogène ou représente $NR^{12}R^{13}$, $R^{12}$ et $R^{13}$ étant identiques ou différents et représentant un atome d'hydrogène ou un groupe alkyle à 1 à 4 atomes de C ;

$R^5$ représente un groupe alkyle à 1 à 10 atomes de C ou cycloalkyle à 3 à 8 atomes de C ayant jusqu'à 8 atomes dans le cycle ou représente un halogène ; et

n est égal à 1 ou 2, à l'exception toutefois de la 2-hydroxy-5-méthyl-3-méthylsulfinylacétophénone, de la 2-hydroxy-5-méthyl-3-méthylsulfonylacétophénone, de l'acide 5-chloro-3-méthylsulfonylsalicyclique, de l'acide 3-bromo-5-méthylsulfonylsalicylique et de l'acide 5-bromo-3-n-butyl-sulfonylsalicyclique, caractérisé en ce que :

a) on fait réagir des composés de formule II

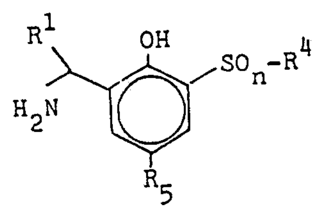

(II)

dans laquelle $R^1$ représente un atome d'hydrogène ou un groupe alkyle à 1 à 4 atomes de C, $R^4$, $R^5$ et n ont les significations indiquées ci-dessus, $R^4$ cependant pouvant aussi représenter un groupement —N = Z, Z ayant la signification d'un groupe protecteur de formule V

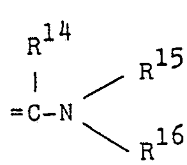

(V)

dans laquelle les radicaux $R^{14}$ désignent un atome d'hydrogène ou un groupe alkyle à 1 à 4 atomes de C et $R^{15}$, $R^{16}$ désignent un groupe alkyle à 1 à 4 atomes de C, avec du formaldéhyde ou un réactif donnant naissance à du formaldéhyde pour obtenir des composés de formule I et éventuellement on sépare le groupe protecteur Z par hydrolyse ;

b) on fait réagir des composés de formule III

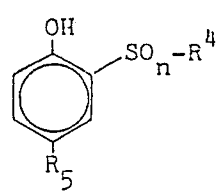

(III)

dans laquelle $R^4$, $R^5$ et n ont les significations indiquées ci-dessus, $R^4$ cependant peut avoir en outre la signification —N = Z définie dans la variante a) du procédé avec un réactif de formylation pour obtenir des composés de formule générale I et éventuellement on sépare le groupe protecteur Z par hydrolyse ;

c) on oxyde des composés de formule générale IV

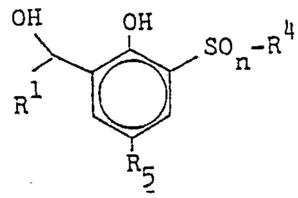

(IV)

dans laquelle $R^1$, $R^4$, $R^5$ et n ont les significations indiquées, $R^4$ cependant pouvant avoir en outre la signification —N = Z définie dans la variante a) du procédé pour obtenir des composés de formule générale I et éventuellement on sépare le groupe protecteur Z par hydrolyse ;

d) on oxyde des composés de formule I dans laquelle $R^1$ représente un atome d'hydrogène, $R^2$ et $R^3$ représentent ensemble la fonction carbonyle, $R^4$ et $R^5$ ont les significations indiquées ci-dessus, $R^4$ cependant pouvant avoir aussi la signification —N = Z définie dans la variante a) du procédé pour obtenir des composés de formule générale I et éventuellement on sépare le groupe protecteur Z par hydrolyse et éventuellement on transforme les acides obtenus en leurs esters ou amides de formule générale I ;

e) on transforme des composés de formule générale I dans laquelle $R^1$ représente un atome d'hydrogène ou un groupe alkyle à 1 à 4 atomes de C ; $R^2$, $R^3$ forment ensemble une fonction carbonyle ; $R^4$, $R^5$ et n ont les significations indiquées ci-dessus, avec des alcools $R^{10}OH$, des thioalcools $R^{10}SH$, des amines $NHR^{10}R^{11}$ ainsi que des composés $HX\text{-}(CH_2)_m\text{—}XH$, $R^{10}$, $R^{11}$, X et m ayant les significations indiquées ci-dessus, en les acétals, les thioacétales, aminales ou imines correspondants de formule générale I ;

f) on chlorosulfone des composés de formule générale XVII

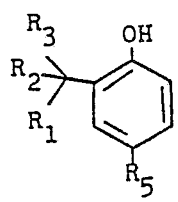

(XVII)

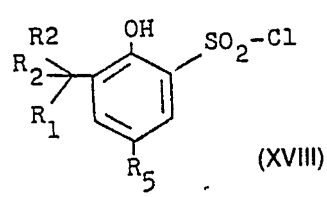

(XVIII)

et on fait réagir les sulfochlorures XVIII formés avec des amines

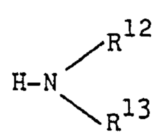

pour donner les composés I ou on les soumet à une réduction pour donner les acides sulfiniques XIX

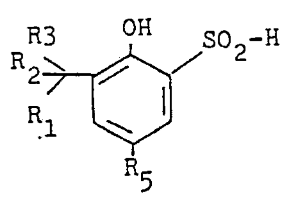

(XIX)

et on transforme ceux-ci par alkylation en les composés I.

2. Procédé selon la revendication 1 caractérisé en ce que

$R^1$ représente un atome d'hydrogène ou OH ;

$R^2$, $R^3$ forment ensemble une fonction carbonyle ou une chaîne

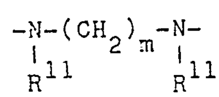

m étant égal à 2 ou 3 et $R^{11}$ signifiant méthyle ou éthyle ;

$R^4$ signifie méthyle ou $NH_2$ ;
$R^5$ signifie un groupe alkyle à 4 à 8 atomes de C, et
n signifie 1 ou 2.

3. Procédé selon la revendication 2 caractérisé en ce que $R^1$ représente un atome d'hydrogène, $R^2$ et $R^3$ représentent ensemble la fonction carbonyle, $R^4$ représente un groupe méthyle, $R^5$ un groupe alkyle à 4 à 8 atomes de C et n est égal à 1 ou 2.

4. Procédé selon la revendication 3 caractérisé en ce que $R^5$ désigne un groupe diméthyléthyle et n égale à 2.

5. Procédé selon la revendication 3 caractérisé en ce que $R^5$ est un groupe 1,1-diméthyléthyle et n est égal à 1.

6. Procédé selon la revendication 1 caractérisé en ce que $R^1$ représente un atome d'hydrogène, $R^2$ et $R^3$ représentent ensemble la fonction carbonyle, $R^4$ représente un groupe chlorométhyle, $R^5$ un groupe 1,1-diméthyléthyle et n est égal à 2.

7. Procédé selon la revendication 2 caractérisé en ce que $R^1$ représente un atome d'hydrogène, $R^2$ et $R^3$ représentent ensemble la fonction carbonyle, $R^4$ représente $NH_2$, $R^5$ un groupe 1,1-diméthyléthyle et n est égal à 2.

8. Procédé selon la revendication 2 caractérisé en ce que $R^1$ représente un atome d'hydrogène, $R^2$ et $R^3$ représentent ensemble

$$-\underset{\displaystyle CH_3}{\underset{|}{N}}-(CH_2)_2-\underset{\displaystyle CH_3}{\underset{|}{N}}-,$$

$R^4$ représente le groupe méthyle, $R^5$ le groupe 1,1-diméthyléthyle et n est égal à 2.

9. Procédé selon la revendication 1 caractérisé en ce que : $R^1$ représente un atome d'hydrogène ou un groupe OH ; $R^2$ et $R^3$ représentent ensemble la fonction carbonyle = O ; $R^4$ signifie $NR^{12}R^{13}$, $R^{12}$ et $R^{13}$ étant identiques ou différents et représentant un atome d'hydrogène ou un groupe alkyle à 1 à 4 atomes de C ; et en ce que $R^5$ représente un groupe alkyle à 3 à 8 atomes de C ; et n est égal à 2.